# EUROPÄISCHE PATENTSCHRIFT

(11) **EP 2 931 286 B1**
(45) Veröffentlichungstag und Bekanntmachung des Hinweises auf die Patenterteilung: **04.10.2017**
(21) Anmeldenummer: 13798323.5
(22) Anmeldetag: 28.11.2013
(51) Int. Cl.: A61K 31/7042, A61K 45/00, A61K 47/40, A61P 35/00, A61K 38/17, A61K 38/19, A61K 31/352, B82Y 5/00

(54) **DELPHINIDIN ZUR BEKÄMPFUNG VON MELANOMZELLEN**
DELPHINIDIN FOR COMBATING MELANOMA CELLS
DELPHINIDIN CONTRE LES CELLULES DE MÉLANOME

(30) Priorität: 11.12.2012 DE 102012222777; 11.01.2013 EP 13150909
(43) Veröffentlichungstag der Anmeldung: 21.10.2015
(73) Patentinhaber: SapioTec GmbH, 97082 Würzburg (DE)
(72) Erfinder: ROEWER, Norbert, 97082 Würzburg (DE); BROSCHEIT, Jens, 97209 Würzburg (DE)
(74) Vertreter: Glawe, Delfs, Moll
(86) Internationale Anmeldenummer: PCT/EP2013/074991
(87) Internationale Veröffentlichungsnummer: WO 2014/090586

(56) Entgegenhaltungen:
- WO-A2-2006/076387
- US-A1- 2005 013 880
- US-A1- 2009 270 348
- Biolink: "Biolink synthetic anthocyanin molecules becomes cancer figthers", , 22. Mai 2009 (2009-05-22), Seiten 1-4, XP002692595, Gefunden im Internet: URL:http://www.biolink.no/biolink/cancer-a rticle95-6.html [gefunden am 2013-02-21]
- AFAQ FARRUKH ET AL: "Delphinidin, an anthocyanidin in pigmented fruits and vegetables, protects human HaCaT keratinocytes and mouse skin against UVB-mediated oxidative stress and apoptosis", JOURNAL OF INVESTIGATIVE DERMATOLOGY, Bd. 127, Nr. 1, Januar 2007 (2007-01), Seiten 222-232, XP002692596, ISSN: 0022-202X
- WANG L S ET AL: "Anthocyanins and their role in cancer prevention", CANCER LETTERS, NEW YORK, NY, US, Bd. 269, Nr. 2, 8. Oktober 2008 (2008-10-08), Seiten 281-290, XP025496527, ISSN: 0304-3835, DOI: 10.1016/J.CANLET.2008.05.020 [gefunden am 2008-06-20]
- SERAFINO ANNALUCIA ET AL: "Differentiation of human melanoma cells induced by cyanidin-3-O-beta-glucopyranoside", FASEB JOURNAL, Bd. 18, Nr. 12, September 2004 (2004-09), Seiten 1-25, XP002692597, ISSN: 0892-6638
- UEDA H ET AL: "EVALUATION OF A SULFOBUTYL ETHER BETA-CYCLODEXTRIN AS A SOLUBILIZING/STABILIZING AGENT FOR SEVERAL DRUGS", DRUG DEVELOPMENT AND INDUSTRIAL PHARMACY, NEW YORK, NY, US, Bd. 24, Nr. 9, 1. September 1998 (1998-09-01), Seiten 863-867, XP009040590, ISSN: 0363-9045, DOI: 10.3109/03639049809088532
- DATABASE BIOSIS [Online] BIOSCIENCES INFORMATION SERVICE, PHILADELPHIA, PA, US; September 2012 (2012-09), VOERSMANN H ET AL: "Three dimensional melanoma treatment using the death ligand TRAIL", XP009176203, Database accession no. PREV201200611221 & JOURNAL OF INVESTIGATIVE DERMATOLOGY, Bd. 132, Nr. Suppl. 2, September 2012 (2012-09), Seite S129, 42ND ANNUAL MEETING OF THE EUROPEAN-SOCIETY-FOR-DERMATOLOGICAL-RESEAR CH (ESDR); VENICE, ITALY; SEPTEMBER 07 -10, 2012 ISSN: 0022-202X(print)

## Beschreibung

Die Erfindung betrifft Zusammensetzungen umfassen
Als eine erste Komponente
- einen Komplex aus Delphinidin und einem Sulfoalkylether-β-Cyclodextrin und/oder
- Delphinidin oder dessen Salze und als eine weitere Komponente
- ein Tumornekrosefaktor-verwandten Apoptose-induzierten Liganden (Tumor Necrosis Factor Related Apoptosis Inducing Ligand - TRAIL)
zur Verwendung bei der Behandlung von malignem Melanom.

Bei dem malignen Melanom, auch unter der Bezeichnung "schwarzer Hautkrebs" bekannt, handelt es sich um eine bösartige Entartung der Pigmentzellen, den sogenannten Melanozyten. Der Krebs neigt bereits in einem frühen Stadium zum Streuen von Metastasen über Blut- und Lymphbahnen. Das maligne Melanom ist heilbar, jedenfalls dann, wenn es früh diagnostiziert und behandelt wird.

Die wichtigste Behandlungsmethode ist die chirurgische Entfernung des Tumors neben der Strahlentherapie, obgleich auch die Monotherapie mit Interferonen bei der Behandlung des malignen Melanoms Anwendung findet. Ebenfalls bekannt ist die Vakzintherapie, d.h. die aktive Immuntherapie mittels Tumorimpfungen unter Verwendung von Tumorvakzinen um die körpereigene Abwehr zur gezielten Bekämpfung von Krebszellen anzuregen. Den Abwehrzellen werden dabei über die verabreichten Vakzine Merkmale des Tumors, (beispielsweise Eiweißmoleküle, die von den Tumorzellen produziert werden oder Zellbruchstücke der Tumorzellen) so präsentiert, dass die Abwehrzellen diese Merkmale als fremd erkennen und körpereigenen Zellen, die diese Merkmale tragen, angreifen. Neben der Vakzintherapie ist die Chemotherapie, d.h. die medikamentöse Behandlung mit chemischen Substanzen, die Tumorzellen schädigen oder hemmen (Zytostatika), ebenfalls von praktischer Relevanz. "Biolink synthetic anthocyanin molecules becomes cancer fighters", 22. Mai 2009 (2009-05-22), Seiten 1-4, Gefunden im Internet:URL:http://www.biolink.no/biolink/ cancer-article95-6.html [gefunden am 2013-02-21] offenbart, dass Cyanidin und Delphinidin das Wachstum von einigen Arten von Krebszellen, i.a. TVM-A12 (Melanom) hemmen. Aufgabe der vorliegenden Erfindung ist es, ein wirksames Mittel als Alternative oder Ergänzung zu aus dem Stand der Technik bekannten Behandlungsmöglichkeiten des malignem Melanoms zur Verfügung zu stellen.

Gelöst wird diese Aufgabe durch die in den unabhängigen Ansprüchen genannten Zusammensetzungen und Verwendungen, wobei vorteilhafte Ausführungsformen der Erfindung in den Unteransprüchen offenbart sind. Dass diese Aufgabe in der Tat gelöst wird, belegen die *in-vitro* Versuchsergebnisse zu der Wirkung der erfindungsgemäßen Zusammensetzungen auf Melanomzellen und Nicht-Krebszellen und die Zellvitalität in den Beispielen 6-12, insbesondere in den Beispielen 9-11 bei denen TRAIL als weitere Komponente verwendet wurde.

Zunächst seien einige im Rahmen der Erfindung verwendete Begriffe erläutert.

Die erfindungsgemäße Zusammensetzung wird zur Behandlung eines Objekts oder Individuums eingesetzt, das am malignem Melanom leidet. Der Begriff "Objekt" umfasst lebende Tiere und Menschen. Der Zweck dieser Behandlung ist die zumindest teilweise Abtötung oder Neutralisation der Tumorzellen.

"Neutralisation" und "Abtötung" bedeuten im Sinne der vorliegenden Erfindung die zumindest teilweise Zerstörung oder Auflösung oder Inaktivierung oder Vermehrung der Tumorzellen.

Die vorliegende Erfindung bezieht sich auch auf Verfahren zur Behandlung eines an malignem Melanom leidenden Objekts, wobei dem Objekt eine therapeutisch wirksame Menge der erfindungsgemäßen Zusammensetzung verabreicht wird. Von besonderen Interesse ist es dabei, einerseits die Melanomzellen mit den Wirkstoff zu bekämpfen, andererseits aber auch weitest möglich die Vitalität und Proliferation der Nicht-Krebszellen unbeeinträchtigt zu lassen.

Der Begriff "Behandlung" bedeutet im Sinne der vorliegenden Erfindung das ganze oder teilweise Erreichen der nachfolgend genannten Ergebnisse: Ganze oder teilweise Reduktion des Krankheitsbildes; Verbesserung mindestens eines der klinischen Symptome oder mit der Krankheit assoziierten Indikatoren; Verzögerung, Unterdrückung oder Schutz vor dem Fortschreiten der Krankheit; oder ganze oder teilweise Verzögerung, Unterdrückung oder Schutz vor dem Ausbrechen oder Entstehung der Krankheit. Das zu behandelnde Objekt ist ein Mensch oder Tier, vorzugsweise ein Säugetier. Die veterinärmedizinische Behandlung umfasst neben der Behandlung von Nutz- oder Wildtieren (z.B. Schafe, Katzen, Pferde, Kühe, Schweine) auch Labortiere (z.B. Ratten, Mäuse, Meerschweine, Affen).

Die weitere therapeutisch wirksame Substanz ist ein Tumornekrosefaktor-verwandter Apoptose-induzierter Ligand (Tumor Necrosis Factor Related Apoptosis Inducing Ligand - TRAIL). Gemäß einer weiteren bevorzugten Ausführungsform der Erfindung ist eine mögliche weitere hinzufügbare therapeutisch wirksame Substanz ausgewählt aus der Gruppe bestehend aus Zytostatika, Interferone, vorzugsweise Alpha- und/oder Beta-Interferone, weiter vorzugsweise Interferon alpha 2-a und alpha 2-b, und Tumorvakzine. Letztere können auch in Kombination mit TRAIL kombiniert und angewendet werden. Insbesondere wenn sich bereits Metastasen in anderen Organen gebildet haben, können eine Kombination von Chemotherapie und Immuntherapie, sowie eine Strahlenbehandlung einzelner Metastasen angebracht sein um Rückbildungen zu erreichen.

Bei der Chemotherapie werden üblicherweise Interferone, insbesondere von den weißen Blutkörperchen (Leukozyten) gebildetes IFN-alpha, und von den Bindegewebszellen (Fibroblasten) gebildetes IFN-beta, verwendet, die das Wachstum von gesunden als auch bösartigen Zellen hemmen und das Immunsystem anregen. In der Krebstherapie wird üblicherweise auf reines, gentechnologisch gewonnenes Interferon zurückgegriffen, beispielsweise auf das in Deutschland zur Krebsbehandlung zugelassene Interferon alpha 2-a (Roferon®) und Interferon alpha 2-b (IntronA®).

Bei der Immuntherapie kommt das ursprüngliche Konzept, bei dem körpereigene Tumorzellen durch Bestrahlung teilungsunfähig gemacht und um den Reiz für das Immunsystem zu erhöhen, vorzugsweise mit einem Virus vermischt, in die Haut gespritzt wird um Abwehrzellen anzulocken und gezielt gegen diese Tumorzellen zu aktivieren, immer seltener zur Anwendung. Statt der Verwendung ganzer Tumorzellen oder Zellbruchstücke wird nunmehr regelmäßig auf von den Tumorzellen produzierte Eiweißmoleküle zurückgegriffen, mit denen aus Blutvorläuferzellen gezüchtete Zellen *in-vitro* beladen werden und so bestückt dem Patienten zurückgegeben werden um sie den Abwehrzellen als etwas zu präsentieren, was bekämpft werden muss. Alternativ kommt man zum gleichen Ergebnis, wenn in die Tumorzellen das Gen eines Lock- bzw. Aktivierungsstoffes für Abwehrzellen eingeschleust wird.

Von praktischer Relevanz ist ebenfalls die eingangs genannte und gegen Krebszellen gerichtete Chemotherapie bei der verschiedene Medikamente verwendet werden, die das Tumorwachstum auf unterschiedliche Weise hemmen können, wobei diese Chemotherapeutika üblicherweise als "Zytostatika" bezeichnet werden. Zytostatika werden synthetisch hergestellt oder sind Abkömmlinge von in der Natur vorkommenden Zellgiften, die den programmierten Zelltod (Apoptose) der Tumorzellen auslösen. Beispiele für chemotherapeutische Mittel, welche im Rahmen der vorliegenden Erfindung Verwendung finden können, schließen Bortezomib (Velcade®, Millennium), Melphatan, Prednison, Vincristin, Carmustin, Cyclophosphamid, Dexamethason, Thalidomid, Doxorubicin, Cisplatin, Etoposid und Cytarabin ein.

Neben der erfindungsgemäßen medikamentösen Verabreichung kann ergänzend eine Strahlentherapie zur Anwendung kommen. Abgesehen von der Verwendung radioaktiver Medikamente, so genannten "Radiopharmaka", wird die Strahlentherapie vorzugsweise örtlich begrenzt eingesetzt. Das heißt, die vorzugsweise elektromagnetische Strahlung und auch Teilchenstrahlen wirken in diesem Fall örtlich begrenzt in dem durchstrahlten Bereich und schädigen die Tumorzellen, insbesondere die DNS im Zellkern durch Ionisation und Entstehung freier Radikale und Brüchen in der DNS der Tumorzellen. Um das den Tumor umgebende gesunde Gewebe zu schützen können Blenden verwendet werden.

Die Verabreichung des erfindungsgemäßen Wirkstoffs im Wege der Kombination mit TRAIL und gegebenenfalls anderen weiteren therapeutischen Wirkstoffen, beispielsweise ausgewählt aus der Gruppe bestehend aus Zytostatika, Interferone und Tumorvakzine, kann auch als so genannte regionale Therapie erfolgen, beispielsweise durch gezielte Einspritzung (z.B. mittels Katheter) in Körperregionen, Körperhöhlen oder in die Blutgefäße der Tumorregion bzw. des Organs, in dem der Tumor sitzt. Eine Durchströmung des betroffenen Bereichs oder des Organs ist auch im Weg einer regionalen Perfusion möglich, bei der das Medikament den Bereich (z.B. Arm oder Bein) oder das Organ unter Abschluss vom übrigen Kreislauf durchströmt und direkt wieder abgeleitet wird ohne in den übrigen Körper zu gelangen.

Die erfindungsgemäße Zusammensetzung wird vorzugsweise als pharmazeutische Zusammensetzung bereitgestellt und verabreicht. Der Begriff "pharmazeutische Zusammensetzung" umfasst ein oder mehrere Wirkstoffe und ein oder mehrere innerte Inhaltstoffe, die als Träger für den Wirkstoff bzw. die Wirkstoffe fungieren. Die pharmazeutischen Zusammensetzungen erlauben es, den erfindungsgemäßen Komplex oder die erfindungsgemäße Zusammensetzung oral, rektal, parenteral, einschließlich intraperitoneal, perkutan, subkutan, intramuskulär, intravenös, ophthalmisch, pulmonal oder nasal zu verabreichen. Eine parenterale Verabreichungsform kann beispielsweise eine Tablette, Kapsel, Lösung, Suspension oder Dispersion sein. Eine ophthalmische, pulmonale oder nasale Verabreichungsform kann beispielsweise ein Aerosol, Lösung, Creme, Paste, Lotion, Gel, Salbe, Suspension oder Dispersion sein. Entsprechende Techniken für die Formulierung und Verabreichung sind aus dem Stand der Technik bekannt, siehe beispielsweise "Remington's Pharmaceutical Sciences" (Mack Publishing Co, Easton Pa.). Zum Beispiel können die erfindungsgemäßen Zusammensetzungen einem Subjekt intravenös mittels eines pharmazeutisch akzeptablen Trägers (z.B. physiologische Salzlösung) verabreicht werden. Für die Injektion bietet sich eine Formulierung in wässriger Lösung, vorzugsweise in physiologisch akzeptablen Puffern an (z.B. Hanks-Lösung, Ringer-Lösung oder physiologisch gepufferte Salzlösung). Für die parenterale Verabreichung, einschließlich der intravenösen, subkutanen, intramuskulären und intraperitonealen Verabreichung, kommt ebenfalls eine wässrige oder ölige Lösung oder eine Feststoffformulierung in Betracht. Der Anteil des aktiven Wirkstoffs in der pharmazeutischen Zusammensetzung kann variieren und liegt üblicherweise zwischen 2 und 60 Gew.-% der Verabreichungseinheit. Der Wirkstoffanteil ist entsprechend so ausgewählt, dass eine wirksame Dosis erreicht wird. Gemäß einer bevorzugten Ausführungsform der Erfindung werden das Delphinidin oder dessen Salze und/oder der Komplex aus Delphinidin und dem Sulfoalkylether-β-Cyclodextrin in einer galenischen Zubereitung zur kontrollierten und/oder zeitverzögerten Freisetzung des Delphinidins eingesetzt.

"Salz" oder "pharmazeutisch akzeptables Salz" steht für jegliches unter pharmazeutischen Gesichtspunkten akzeptables Salz einer Verbindung vorliegender Erfindung, welches den pharmazeutisch wirksamen Wirkstoff oder dessen aktives Metabolit nach der Verabreichung freigeben kann. Salze der Zusammensetzungen und Komplexe der vorliegenden Erfindung können von anorganischen oder organischen Säuren und Basen abgeleitet sein.

Das Anthocyanidin Delphinidin kann in "Reinform" oder "gereinigt" verwendet werden, was bedeutet, dass ungewünschte Komponenten entfernt wurden.

"Anthocyanidine" weisen die nachfolgend wiedergegebene Grundstruktur auf.

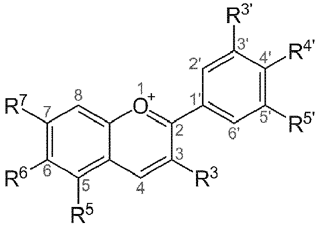

Die Substituenten in dieser Formel sind ausgewählt aus der Gruppe bestehend aus Wasserstoff, Hydroxygruppe und Methoxygruppe.

Cyclodextrine, die mit dem Anthocyanidin Delphinidin erfindungsgemäß komplexiert sein können, sind zyklische Oligosaccharide aus α-1,4-glykosidisch verknüpften Glukosemolekülen. β-Cyclodextrin besitzt sieben Glukoseeinheiten. Bei einem Sulfoalkyether-β-Cyclodextrin sind Hydroxygruppen der Glukoseeinheit in einem Sulfoalkylalkohol verethert. Erfindungsgemäß ist in der Regel lediglich ein Teil der 21 Hydroxygruppen eines β-Cyclodextrins verethert. Die Herstellung von Sulfoalkyethercyclodextrinen ist dem Fachmann geläufig und beispielsweise in US 5,134,127 oder WO 2009/134347 A2 beschrieben.

Sulfoalkyethergruppen werden bei Cyclodextrinen im Stand der Technik zur Erhöhung der Hydrophilie beziehungsweise Wasserlöslichkeit eingesetzt. Sulfoalkylethergruppen tragen in besonderem Maße zur Erhöhung der Stabilität des Komplexes aus Anthocyanidinen und dementsprechend substituiertem β-Cyclodextrin bei, so dass damit die Lagerstabilität und Formulierbarkeit der besonders oxidationsempfindlichen Anthocyanidine wesentlich verbessert wird. Der erfindungsgemäße Komplex kann als lagerstabile wässrige Lösung oder Feststoff formuliert werden, wie unten noch näher gezeigt werden wird.

Erfindungsgemäß besonders bevorzugt ist die Komplexierung des Wirkstoffs Delphinidin mit einem Sulfoalkylether-β-Cyclodextrin, vorzugsweise einem Sulfobutylether-β-Cyclodextrin (SBE-β-CD) oder einem Sulfoethylether-β-Cyclodextrin, was überraschenderweise die Löslichkeit und Stabilität des Wirkstoffs erhöht. Ein den Schutzbereich nicht beschränkender Erklärungsversuch hierfür ist, dass die negativ geladenen Sulfobutyleinheiten bzw. Sulfoethyleinheiten mit den positiv geladenen Anthocyanidin Delphinidin elektrostatisch Wechselwirken und unter den Alkylgruppen die Butylgruppe bzw. Ethylgruppe die optimale Länge besitzen, um sterisch eine entsprechende Wechselwirkung zu ermöglichen. An dieser Stelle sei angemerkt, dass keine allgemein gültige Aussage getroffen werden kann, dass ein beliebiger Wirkstoff, beispielsweise Delphinidin, im Komplex mit einem Sulfoalkylether-β-Cyclodextrin zu einer Verbesserung der Löslichkeit und Stabilität führt. Beispielhaft verwiesen sei an dieser Stelle auf Tabelle 1 in Ueda et al., "Evaluation of a Sulfobutyl Ether β-Cyclodextrin as a Solubizing/Stabilizing Agent for Several Drugs", Drug Development and Industrial Pharmacy, 24(9), 863-867 (1998), in der die Löslichkeiten verschiedener Wirkstoffe allein, im Komplex mit Sulfobotylether-β-Cyclodextrin und im Komplex mit β-Cyclodextrin gegenübergestellt sind. Aus den dort dargestellten Löslichkeitswerten (SBE7-β-CD vs. β-CD) erkennt man, dass bei einem Drittel der untersuchten Wirkstoffe im Komplex mit Sulfobotylether-β-Cyclodextrin genau das Gegenteil der Fall ist, d.h. der Komplex aus Wirkstoff und Sulfobotylether-β-Cyclodextrin eine signifikant geringere Löslichkeit gegenüber dem Komplex mit β-Cyclodextrin zur Folge hat.

Bevorzugt beträgt der Substitutionsgrad des Cyclodextrins mit Sulfoalkylethergruppen 3 bis 8, weiter vorzugsweise 4 bis 8, weiter vorzugsweise 5 bis 8, weiter vorzugsweise 6 bis 7. Geeignete Sulfobutylether-β-Cyclodextrine mit einem mittleren Substitutionsgrad von 6 bis 7 sind beispielsweise in der WO 2009/134347 A2 beschrieben und unter dem Handelsnamen Captisol® kommerziell erhältlich. Ebenfalls verwendbar sind entsprechende Cyclodextrine mit einem Substitutionsgrad von 4 bis 5, beispielsweise 4,2.

Das erfindungsgemäß in reiner, Salz- oder komplexierten Form verwendete Anthocyanidin ist Delphinidin. Die chemische Struktur entspricht der oben wiedergegebenen Formel mit dem folgenden Substitutionsmuster

| | R^{3'} | R^{4'} | R^{5'} | R³ | R⁵ | R⁶ | R⁷ |
|---|---|---|---|---|---|---|---|
| Delphinidin | -OH | -OH | -OH | -OH | -OH | -H | -OH |

Gegenstand der Erfindung ist ferner eine wässrige Lösung der erfindungsgemäßen Zusammensetzung zur Verwendung als Medikament zur Behandlung von malignem Melanom.

Die Herstellung des erfindungsgemäßen Komplexes sowie einer entsprechend wässrigen Lösung umfasst die folgenden Schritte:
a) Herstellen einer wässrigen Lösung des Sufoalkylether-β-Cyclodextrins,
b) Zugabe des Anthocyanidin Delphinidin und Vermischen zur Herstellung des Komplexes.

In Schritt a) wird bevorzugt eine wässrige Lösung hergestellt, die 5 bis 10 Gew.-% des verwendeten Cyclodextrins enthält. Besonders bevorzugt ist es im Rahmen der Erfindung, wenn der pH-Wert der wässrigen Lösung während oder nach, bevorzugt jedoch vor der Zugabe des Delphinidins auf einen pH-Wert von 7 oder weniger, vorzugsweise 6 oder weniger, weiter vorzugsweise 5 oder weniger, weiter vorzugsweise 4 bis 5, eingestellt wird. Es hat sich gezeigt, dass bei diesem pH-Wert sich eine höhere Konzentration des Komplexes in wässriger Lösung einstellen lässt.

Die Konzentration des Delphinidins berechnet als Chlorid, beträgt vorzugsweise wenigstens 0,5 mg/ml, weiter vorzugsweise wenigstens 1,0 mg/ml, weiter vorzugsweise wenigstens 1,5 mg/ml, weiter vorzugsweise 2,0 mg/ml. Der besonders bevorzugte Konzentrationsbereich von wenigstens 2,0 mg/ml lässt sich im Rahmen einer bevorzugten Ausführungsform insbesondere einstellen in einer wässrigen Lösung mit einem pH-Wert zwischen 4 und 5.

Im Rahmen der Herstellung kann das Vermischen der Bestandteile der wässrigen Lösung durch Rühren geschehen, bevorzugte Zeiträume für das Vermischen sind 2 bis 20 h. Bevorzugt wird im Dunkeln gearbeitet, um eine lichtinduzierte Oxidation zu vermeiden.

Ein weiterer Gegenstand der Erfindung ist ein Feststoff zur Verwendung als Medikament bei der Behandlung von malignem Melanom, der erfindungsgemäß erhältlich ist durch Entfernen des Lösungsmittels aus einer oben beschriebenen erfindungsgemäßen wässrigen Lösung. Das Entfernen kann bevorzugt durch Gefriertrocknung (Lyophilisation) erfolgen. Sowohl die erfindungsgemäße wässrige medikamentöse Lösung als auch der medikamentöse Feststoff besitzen eine hohe Lagerstabilität.

Die Erfindung soll nun im Folgenden weiter in den Beispielen unter Bezug auf die beigefügten Figuren beschrieben werden, ohne auf diese beschränkt zu sein.
- Figur 1: zeigt Histogramme von mit Delphinidin-SBE-β-CD behandelten (b) und unbehandelten (a) Zellen der humanen Melanom-Zelllinie A-375.
- Figur 2a: zeigt die mittels sub-G1-Peak-Methode ermittelte dosisabhängige Apoptoseinduktion durch Delphinidin-SBE-β-CD und Delphinidin Cl im Vergleich zu den Kontrollen SBE-β-CD, DMSO sowie unbehandelten A-375 Zellen bei niedriger Zellkonfluenz.
- Figur 2b: zeigt die mittels sub-G1-Peak-Methode ermittelte dosisabhängige Apoptoseinduktion von Delphinidin-SBE-β-CD im Vergleich zu Delphinidin Cl und den Kontrollen SBE-β-CD, DMSO sowie unbehandelten A-375 Zellen bei hoher Zellkonfluenz.
- Figur 3a: zeigt die mittels WST-1-Assays ermittelte dosisabhängige Zellviabilitätswirkung von Delphinidin-SBE-β-CD und Delphinidin Cl im Vergleich zu den Kontrollen SBE-β-CD, DMSO sowie unbehandelten A-375 Zellen bei niedriger Zellkonfluenz.
- Figur 3b: zeigt die mittels WST-1-Assays ermittelte dosisabhängige Zellviabilitätswirkung von Delphinidin-SBE-β-CD im Vergleich zu Delphinidin Cl und den Kontrollen SBE-β-CD, DMSO sowie unbehandelten A-375 Zellen bei hoher Zellkonfluenz.
- Figur 4: zeigt mikroskopische Aufnahmen von unbehandelten und mit Delphinidin-SBE-β-CD, Delphinidin Cl sowie SBE-β-CD behandelten A-375 Zellen.
- Figur 5: zeigt Zellzahl und Zellproliferation (EchtzeitZell-Monitoring) von mit Delphinidin-SBE-β-CD behandelten A-375 Zellen hoher Zellkonfluenz im Vergleich zu unbehandelten Zellen mit dem System XCELLLigence.
- Figur 6: zeigt Zellzahl und Zellproliferation (EchtzeitZell-Monitoring) von mit Delphinidin-SBE-β-CD behandelten A-375 Zellen niedriger Zellkonfluenz im Vergleich zu unbehandelten Zellen mit dem System XCELLLigence.
- Figur 7: zeigt Zellzahl und Zellproliferation (EchtzeitZell-Monitoring) von mit Delphinidin Cl behandelten A-375 Zellen hoher Zellkonfluenz im Vergleich zu unbehandelten Zellen mit dem System XCELLLigence.
- Figur 8: zeigt Zellzahl und Zellproliferation (EchtzeitZell-Monitoring) von mit Delphinidin Cl behandelten A-375 Zellen niedriger Zellkonfluenz im Vergleich zu unbehandelten Zellen mit dem System XCELLLigence.
- Figur 9: zeigt Zellzahl und Zellproliferation (EchtzeitZell-Monitoring) von mit SBE-β-CD behandelten A-375 Zellen hoher Zellkonfluenz im Vergleich zu unbehandelten Zellen mit dem System XCELLLigence.
- Figur 10: zeigt Zellzahl und Zellproliferation (EchtzeitZell-Monitoring) von mit SBE-β-CD behandelten A-375 Zellen niedriger Zellkonfluenz im Vergleich zu unbehandelten Zellen mit dem System XCELLLigence.
- Figur 11a: zeigt die mittels sub-G1-Peak-Methode ermittelte Apoptoseinduktion durch Delphinidin-SBE-β-CD mit und ohne TRAIL im Vergleich zu den Kontrollen (unbehandelte Zellen bzw. nur mit TRAIL behandelte Zellen) bei niedriger Zellkonfluenz.
- Figur 11b: zeigt die mittels sub-G1-Peak-Methode ermittelte Apoptoseinduktion durch Delphinidin-SBE-β-CD mit und ohne TRAIL im Vergleich zu den Kontrollen (unbehandelte Zellen bzw. nur mit TRAIL behandelte Zellen, mit SBE-β-CD behandelte Zellen) bei hoher Zellkonfluenz.
- Figur 12a: zeigt die mittels WST-1-Assays ermittelte Zellviabilitätswirkung von Delphinidin-SBE-β-CD mit TRAIL im Vergleich zu Delphinidin-SBE-β-CD allein und den Kontrollen (unbehandelte Zellen bzw. nur mit TRAIL behandelte Zellen) bei niedriger Zellkonfluenz.
- Figur 12b: zeigt die mittels WST-1-Assays ermittelte Zellviabilitätswirkung von Delphinidin-SBE-β-CD mit TRAIL im Vergleich zu Delphinidin-SBE-β-CD allein und den Kontrollen (unbehandelte Zellen bzw. nur mit TRAIL behandelte Zellen, mit SBEβ-CD behandelte Zellen) bei hoher Zellkonfluenz.
- Figur 13: zeigt mikroskopische Aufnahmen von unbehandelten, nur mit TRAIL behandelten, nur mit Delphinidin-SBE-β-CD behandelten, sowie mit Delphinidin-SBE-β-CD und TRAIL behandelten A-375 Zellen.
- Figur 14: zeigt Zellzahl und Zellproliferation (EchtzeitZell-Monitoring) von mit TRAIL, DelphinidinSBE-β-CD, TRAIL und Delphinidin-SBE-β-CD behandelten A-375 Zellen hoher Zellkonfluenz im Vergleich zu unbehandelten Zellen mit dem System XCELLLigence.
- Figur 15: zeigt Zellzahl und Zellproliferation (EchtzeitZell-Monitoring) von mit TRAIL, Delphinidin Cl, TRAIL und Delphinidin Cl behandelten A-375 Zellen hoher Zellkonfluenz im Vergleich zu unbehandelten Zellen mit dem System XCELLLigence.
- Figur 16: zeigt Zellzahl und Zellproliferation (EchtzeitZell-Monitoring) von mit TRAIL (0,02 µg/ml), SBE-β-CD (30 µg/ml), TRAIL(0,02 µg/ml) und SBEβ-CD (30 µg/ml) behandelten A-375 Zellen hoher Zellkonfluenz im Vergleich zu unbehandelten Zellen mit dem System XCELLLigence.
- Figur 17: zeigt Zellzahl und Zellproliferation (EchtzeitZell-Monitoring) von mit TRAIL(0,02 µg/ml), SBE-β-CD (1000 µg/ml), TRAIL (0,02 µg/ml) und SBE-β-CD (1000 µg/ml) behandelten A-375 Zellen hoher Zellkonfluenz im Vergleich zu unbehandelten Zellen mit dem System XCELLLigence.
- Figur 18: zeigt die Wirkung von DelphinidinCl und des Delphinidin-SBE-β-CD Komplexes in Konzentrationen von 0,1 µM, 3,2 µM und 100 µM im Vergleich zu wirkstofffreiem Kontrollmedium auf die Vitalität von Endothelzellen unter Anwendung des ATP-Lumineszenz-Assays.

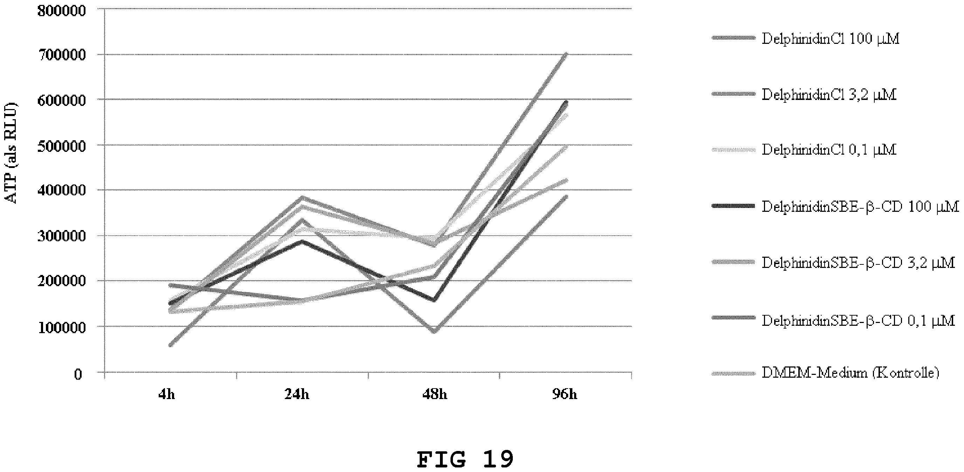

- Figur 19: zeigt die Wirkung von DelphinidinCl und des Delphinidin-SBE-β-CD Komplexes in Konzentrationen von 0,1 µM, 3,2 µM und 100 µM im Vergleich zu wirkstofffreiem Kontrollmedium auf die Vitalität von humanen Fibroblasten unter Anwendung des ATP-Lumineszenz-Assays.

### Beispiele

### I. Herstellung eines Komplexes aus Delphinidin und Cyclodextrinen

### 1. Eingesetzte Materialien:

Die nachfolgenden Cyclodextrine werden verwendet:

| | |
|---|---|
| α-CD | ID No: CYL-2322 |
| β-CD | ID No: CYL-3190 |
| γ-CD | ID No: CYL-2323 |
| (2-Hydroxypropyl)-β-CD | ID No: L-043/07 |
| Sulfobutylether-β-CD | ID No: 47K010111 |

Delphinidinchlorid wurde von der Firma Extrasynthese erworben.

### 2. Bestimmung des Delphinidingehalts

Zur Bestimmung des Gehalts von Delphinidinchlorid in den delphinidinhaltigen Zusammensetzungen wurde ein Reversephase HPLC-Verfahren verwendet. Dabei wurden folgende Reagenzien eingesetzt:
Gereinigtes Wasser
Methanol für die Chromatographie
Ameisensäure, p. a.
1 M Salzsäure als volumetrische Lösung.

Als Säule wurde eine Waters X Bridge™ C18, 35 µl, 150 mm x 4,6 mm verwendet.

Die mobilen Phasen waren wie folgt:
Kanal A: Wasser 950 ml, Methanol 50 ml, Ameisensäure 10 ml
Kanal B: Wasser 50 ml, Methanol 950 ml, Ameisensäure 10 ml

Folgendes Gradientenprogramm wurde verwendet:

| Zeit [min] | Prozent Kanal B |
|---|---|
| 0 | 0 |
| 5 | 0 |
| 25 | 60 |
| 30 | 100 |

Stoppzeit: 35 min
Nachlaufzeit (posttime): 8 min
Flussrate: 1 ml/min
Injektionsvolumen: 20 µl
Säulentemperatur: 30°C +/- 2°C
UV-Vis Detektor: 530 µm für den Assay, 275 µm zur Detektion von Verunreinigungen
Integrator: Fläche

Lösungen und Probenvorbereitung:
Verdünnungslösung 1: Mischung aus 100 ml Methanol und 2,6 ml M HCL
Verdünnungslösung 2: Mischung aus 100 ml 40 prozentiger Methanol und 2,6 ml 1 M HCL

Kalibrierungslösung: Eine Referenzlösung von Delphinidin wurde durch Einwiegen von 10 mg Delphinidinchlorid in einen 10 ml Kolben und Lösen in Verdünnungslösung 1 hergestellt. Nach dem Lösen wurde ungefähr 10fach verdünnt mit Verdünnungslösung 2 zur Herstellung einer ungefähren Konzentration von 0,1 mg/ml.

Die Kontrollkalibrierungslösung wurde auf die gleiche Art und Weise hergestellt. Die Kalibrierungslösungen wurden sofort mittels HPLC analysiert, da Delphinidinchlorid in Lösung instabil ist.

### Herstellung der Prüflösungen:

Zur Bestimmung des Delphinidingehalts erfindungsgemäß hergestellter Feststoffe (Herstellung siehe weiter unten) wurden etwa 50 mg dieser Zusammensetzung in einem 10 ml Kolben eingewogen. Anschließend wurde in Verdünnungslösung 2 gelöst und mit der gleichen Verdünnungslösung 2 weiter verdünnt bis zur Einstellung einer ungefähren Delphinidinkonzentration von 0,1 mg/ml.

Die Bestimmung des Delphinidingehalts in den Proben wurde berechnet unter Zuhilfenahme der Agilent ChemStation Software unter Verwendung der Kalibrierung mit dem beschriebenen externen Standard.

### Beispiel 1: Komplexierung von Delphinidin mit SBE-β-CD

In diesem Beispiel wird die Komplexierung von Delphinidin durch verschiedene Cyclodextrine und die Löslichkeit des Komplexes in wässriger Lösung untersucht.

Es wurden neutrale wässrige Lösungen hergestellt, die 10 Gew.-% des jeweiligen Cyclodextrins enthalten. Bei β-CD wurde aufgrund der mangelnden Löslichkeit eine Konzentration von lediglich 2 Gew.-% gewählt.

Je 5ml der wässrigen Cyclodextrinlösungen und von reinem Wasser wurden in Glaskolben gefüllt. Anschließend wurde ein Überschuss Delphinidinchlorid zugegeben. Die erforderliche Überschussmenge war 10 mg für die Lösungen von α-, β- und γ-Cyclodextrin, und 15 mg für die Lösungen von HPBCD (2-Hydroxypropyl-β-Cyclodextrin) und SBE-β-CD.

Die Suspensionen wurden für 20 h bei 30° C im Dunkeln gerührt. Anschließend wurde filtriert durch einen Membranfilter mit 0,22 µm Porengröße.

Die erzielbaren Löslichkeiten sind in der nachfolgenden Tabelle wiedergegeben.

| Cyclodextrin | Cyclodextrin-Konzentration | Delphinidinchlorid |
|---|---|---|
| - | 0 | 0.07 mg/ml |
| α-CD | 10 % | 0.14 mg/ml |
| β-CD | 2 % | 0.05 mg/ml |
| γ-CD | 10 % | 0.21 mg/ml |
| HPBCD | 10 % | 0.19 mg/ml |
| SBE-β-CD | 10 % | 0.66 mg/ml |

Man erkennt, dass die Komplexierung und die dadurch bewirkte Löslichkeitserhöhung für SBE-β-CD weit besser ist als für die anderen Cyclodextrine.

### Beispiel 2: Einfluss des pH-Wertes

In diesem Beispiel wurde der Einfluss des pH-Wertes auf die Löslichkeit eines Delphinidin-SBE-β-CD in wässriger Lösung untersucht. Nach der Vorschrift von Beispiel 1 wurden wässrige Lösungen von SEB-β-CD hergestellt, diese Lösungen jedoch mit 1 M HCL auf die in Tabelle 2 genannten sauren pH-Werte eingestellt. Anschließend wurde Delphinidinchlorid nach der Vorschrift des Beispiels 1 zugegeben und weitergearbeitet, als einzige Abweichung wurde die Rührzeit auf 2,5 h begrenzt. Die Ergebnisse sind in der nachfolgenden Tabelle wiedergegeben.

| pH | Delphinidinchlorid |
|---|---|
| 6.0 | 0.60 mg/ml |
| 4.8 | 2.12 mg/ml |
| 4.1 | 2.03 mg/ml |

Man erkennt, dass bei pH-Werten zwischen 4 und 5 die Löslichkeit des komplexierten Delphinidinchlorids sich etwa um den Faktor 3 gegenüber einem neutralen pH-Wert erhöht.

### Beispiel 3 Herstellung eines erfindungsgemäßen Feststoffs

In diesem Beispiel wird ein erfindungsgemäßer Komplex als Feststoff formuliert. Zu Vergleichszwecken werden ein Delphinidin/HPBCD Komplex sowie eine Delphinidin/Stärkeformulierung als Feststoff zubereitet.

### Beispiel 3.1: Delphinidin-SBE-β-CD

5 g SEB-β-CD wurden in 40 ml destilliertem Wasser zu einer klaren Lösung gelöst. Der pH-Wert der Lösung wurde mittels 1 M HCL auf 4,8 eingestellt. Anschließend wurden 0,11 g Delphinidinchlorid hinzugefügt und 2 h bei 27°C im Dunkeln gerührt. Die homogene Flüssigkeit wurde durch einen Cellulosenitratmembranfilter mit einer Porengröße von 0,45 µm vakuumfiltriert. Die Lösung wurde gefroren und anschließend gefriergetrocknet bei -48°C und einem Druck von etwa 10,3 Pa (77 mTorr). Das Lyophilisat wurde gemahlen und durch ein Sieb von 0,3 mm Maschenweite gesiebt.

### Beispiel 3.2: Delphinidin HPBCD

Es wurde in gleicher Weise wie Beispiel 3.1 gearbeitet, jedoch wurde bei der Filtration eine signifikante Menge Material abfiltriert, was darauf hindeutet, dass die Solubilisierung deutlich weniger effektiv war als bei Verwendung von SBE-β-CD gemäß Beispiel 3.1.

### Beispiel 3.3 Delphinidin Stärkeformulierung

5 g Stärke wurde in 40 ml destilliertem Wasser suspendiert. Man erhielt eine weiße Suspension. Der pH-Wert der Lösung wurde mit 1 M HCL auf 4,6 eingestellt. Anschließend wurde 0,11 g Delphinidinchlorid zugegeben und für 2 h bei 27° C im Dunkeln gerührt. Die erhaltene homogene Flüssigkeit wurde wie in Beispiel 3.1 gefriergetrocknet, gemahlen und gesiebt.

Beispiel 3.1 ist erfindungsgemäß, bei den Beispielen 3.2 und 3.3 handelt es sich um Vergleichsbeispiele.

### Beispiel 4: Stabilitätsversuche

Die Feststoffe gemäß den Bespielen 3.1 bis 3.3 wurden unter folgenden Bedingungen gelagert:
- 8 Tage bei Raumtemperatur in braunen, verschraubten Glasbehältern,
- anschließend 22 Tage bei Raumtemperatur in Glasbehältern unter Sauerstoffatmosphäre im Dunkeln.

Die letzen 22 Tage der oben beschriebenen Lagerung wurden in Glasviolen mit einem Volumen von 20 ml durchgeführt. Jeweils 250 mg der vorher bereits für 8 Tage gelagerten Proben wurden dort eingefüllt, die Violen wurden mit einem Gummistopfen verschlossen und abgedichtet. Mittels zwei Injektionsnadeln wurde der Kopfraum der Violen mit reinem Sauerstoff gespült. Die Proben wurden anschließend im Dunkeln gelagert.

Der Delphinidingehalt der Feststoffe (berechnet als Delphinidinchlorid und angegeben in Gew.-%) wurde mittels der oben beschriebenen HPLC-Methode bestimmt. Die Ergebnisse finden sich in der nachfolgenden Tabelle.

| | Zeitablauf [Tage] | | | | |
|---|---|---|---|---|---|
| | Start | 2 | 8 | 19 | 30 |
| Beispiel 3.1 | 1.69 | 1.52 | 1.55 | 1.40 | 0.93 |
| Beispiel 3.2 | 1.30 | 1.20 | 1.14 | 1.03 | 0.68 |
| Beispiel 3.3 | 1.60 | 1.59 | 1.56 | 1.53 | 1.15 |

Die Ergebnisse zeigen, dass sich erfindungsgemäß ein Delpinidinkomplex herstellen lässt, der selbst unter reiner Sauerstoffatmosphäre eine hohe Stabilität und damit gute Lagerfähigkeit besitzt. Der Komplex besitzt ferner eine gute Löslichkeit in wässrigen, insbesondere leicht sauren Lösungen, sodass sich Delphinidin erfindungsgemäß auf vielfältige Art und Weise formulieren lässt. Die Stabilität des erfindungsgemäßen Feststoffes ist ähnlich gut wie eine Formulierung mit Stärke (Beispiel 3.3), dieses Vergleichsbeispiel lässt sich allerdings nicht in einer wässrigen Lösung formulieren.

### Beispiel 5: Stabilitätsversuche in wässriger Lösung

Zur Bestimmung des Gehalts von Delphinidinchlorid in den delphinidinhaltigen Lösungen wurde ein Reverse Phase HPLC-Verfahren ähnlich dem oben bereits beschriebenen verwendet. Dabei wurden folgende Reagenzien eingesetzt:
Gereinigtes Wasser
Methanol für die Chromatographie
Armeisensäure, p. a.
1 M Salzsäure als volumetrische Lösung.

Als Säule wurde eine Waters X Bridge™ C18, 35 µl, 150 mm x 4,6 mm verwendet.

Die mobilen Phasen waren wie folgt:
Kanal A: Wasser 770 ml, Methanol 230 ml, Armeisensäure 10 ml
Kanal B: Wasser 50 ml, Methanol 950 ml, Armeisensäure 10 ml

Folgendes Gradientenprogramm wurde verwendet:

| Zeit [min] | Prozent Kanal B |
|---|---|
| 0 | 0 |
| 5 | 0 |
| 20 | 20 |
| 25 | 100 |

Stoppzeit: 25 min
Nachlaufzeit (posttime): 8 min
Flussrate: 1 ml/min
Injektionsvolumen: 20 µl
Säulentemperatur: 30°C +/- 2°C
UV-Vis Detektor: 530 µm für den Assay, 275 µm zur Detektion von Verunreinigungen
Integrator: Fläche

Lösungen und Probenvorbereitung:
Verdünnungslösung 1: Mischung aus 100 ml Methanol und 2,6 ml 1 M HCL
Verdünnungslösung 2: Mischung aus 100 ml 50 %igem Methanol und 2,6 ml 1 M HCL

Kalibrierungslösung: Eine Referenzlösung von Delphinidin wurde durch Einwiegen von 10 mg Delphinidinchlorid in einen 10 ml Kolben und Lösen in Verdünnungslösung 1 hergestellt. Nach dem Lösen wurde ungefähr 10fach verdünnt mit Verdünnungslösung 2 zur Herstellung einer ungefähren Konzentration von 0,1 mg/ml.

Die Kontrollkalibrierungslösung wurde auf die gleiche Art und Weise hergestellt. Die Kalibrierungslösungen wurden sofort mittels HPLC analysiert, da Delphinidinchlorid in Lösung instabil ist.

### Herstellung der Prüflösungen:

Zur Bestimmung des Delphinidingehalts einer erfindungsgemäßen wässrigen Lösung wurden Delphinidin/SBE-β-CD des Beispiels 3.1 (erfindungsgemäß) und Delphinidin (Vergleichsbeispiel in 0,9 %iger NaCl-Lösung gelöst bis zur Einstellung einer Startkonzentration (bezogen auf das Delphinidin) von 1,584 mg/ml (erfindungsgemäßes Beispiel) bzw. 0,0216 mg/ml (Vergleichsbeispiel). Die Lösungen wurden bei Raumtemperatur hergestellt und anschließend bei 37°C im Dunkeln in verschlossenen Violen gelagert.

Nach 1, 2, 3 und 4 h wurde der Delphinidingehalt bestimmt. Die nachfolgende Tabelle gibt den ermittelten Gehalt als Prozentanteil der oben genannten Startkonzentration an.

| Zeit [h] | Delphinidin unkomplexiert | Delphinidin/SBE-β-CD |
|---|---|---|
| 0 | 100% | 100% |
| 1 | 8,3% | 80,7% |
| 2 | 6,5% | 74,5% |
| 3 | 5,6% | 64,7% |
| 4 | 5,1% | 62,8% |

Die Bestimmung des Delphinidingehalts in den Proben wurde berechnet unter Zuhilfenahme der Agilent ChemStation Software unter Verwendung der Kalibrierung mit den beschriebenen externen Standard.

### II. Wirkung von Delphinidin und des Delphinidin-SBE-β-CD Komplexes auf Melanomzellen

### Testzelllinie

In den nachfolgend beschriebenen *in-vitro* Versuchen wurde die Wirkung des Komplexes aus Delphinidin und Sulfobutylether-β-Cyclodextrin (nachfolgend Delphinidin-SBE-β-CD) und Delphinidin auf die exemplarisch verwendete humane Melanom-Zelllinie A-375 [ATTC Katalog Nr. CRL-1619; Bruggen J., Sorg C. (1983) Detection of phenotypic differences on human malignant melanoma lines and their variant sublines with monoclonal antibodies. Cancer Immunol Immunother 15: 200-205] untersucht.

Die Zelllinie wurde bei 37°C, 5% CO₂ in DMEM (Dulbecco's Modified Eagle's Medium von Gibco, Karlsruhe, Deutschland) ergänzt mit 10% FCS (Fetal Bovine Serum) und Antibiotika kultiviert.

### Beispiel 6

### (Apoptoseinduktionsmessung mit Hilfe der sub-G₁-Peak-Methode)

In dem Experiment gemäß Beispiel 6 wurde die Wirkung der untersuchten Substanzen auf die Apoptoseinduktion bei der Testzelllinie mit Hilfe der sub-G₁-Peak-Methode, die auch unter Bezeichnung "Nicoletti-Methode" in der Fachliteratur geläufig ist, untersucht [Riccardi C. Nicoletti I. (2006) Analysis of apoptosis by propidium iodide staining and flow cytometry. Nat. Protoc 1: 1458-1461].

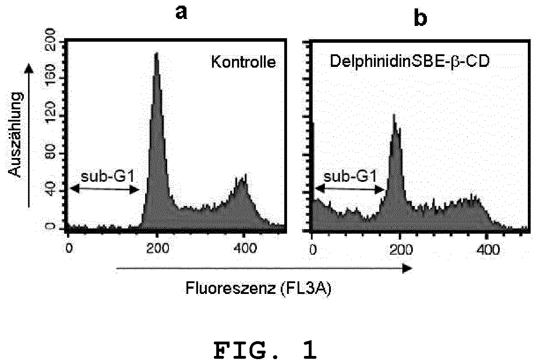

Die Methode basiert auf dem geringeren DNA-Gehalt apoptotischer Zellen im Vergleich zu vitalen Zellen. Ein Merkmal der Apoptose ist die Spaltung der DNA durch Endonukleasen in kurze DNA-Fragmente, wodurch in apoptotischen Zellen im Vergleich zu vitalen Zellen der Gehalt an niedrigmolekularer DNA erhöht und der Anteil an hochmolekularer DNA reduziert ist. Bei induzierter Lyse (Permeabilisierung) der Zellmembran treten die niedermolekularen DNA Fragmente aus den apoptotischen Zellen aus. Werden die Zellen mit Propidiumiodid (PI), einem in die DNA-interkalierenden Farbstoff, gefärbt, fluoreszieren apoptotische Zellen weniger intensiv als vitale Zellen und der durchflusszytometrisch bestimmte DNA-Gehalt erscheint bei apoptotischen Zellkernen als breiter, hypodiploider, schwächer-fluoreszierender DNA-Peak (sub-G₁-Peak), der leicht vom schmalen DNA-Doppelpeak gesunder Zellen mit diploidem DNA-Gehalt unterschieden werden kann. Ein Beispiel dafür ist mit den in Figur 1 dargestellten Histogrammen a und b gegeben. Histogramm a zeigt unbehandelte, PI-gefärbte Zellen. Propidiumiodid hat sich in die DNA eingelagert und fluoresziert in zwei schmalen Peaks, die Zellen der G1-Phase (linker Peak) mit einfachem und Zellen in der G2-Phase (rechter Peak) mit doppeltem DNA-Gehalt. Ein dem linken Peak vorgelagerter sub-G₁-Peak fehlt nahezu. Histogramm b zeigt dagegen einen breiten sub-G₁-Peak. Dies stellt den Anteil apoptotischer Zellkerne nach 24 Stunden Delphinidin-SBE-β-CD-Behandlung dar.

Für die Durchführung der sub-G1-peak-Methode gemäß Beispiel 6 wurden die Zellen für 24 Stunden mit 10-3000 µg Delphinidin-SBE-β-CD pro ml Zellsuspension und 15-120 µM gereinigtes Delphinidinchlorid (nachfolgend Delphinidin Cl) pro ml Zellsuspension inkubiert, wobei mit dem Komplexpartner SBE-β-CD sowie DMSO behandelte Zellen, als auch unbehandelte Zellen als Kontrollen dienten. Für jeden Einzelversuch (Kontrolle, Delphinidin-SBE-β-CD, SBE-β-CD, Delphinidin Cl) wurden drei Vertiefungen a 3 ml auf einer Well-platte mit Zellen angesetzt. Anschließend wurden die Zellen mittels Trypsinierung geerntet, mit eiskalter Phosphatgepufferter Salzlösung (PBS) gewaschen und für 1 Stunde mit dem Färbungspuffer enthaltend 0,1% Natriumzitrat, 0,1% Triton X-100 und PI (40µg/ml; Sigma-Aldrich, Taufkirchen, Deutschland) inkubiert, bevor anschließend der DNA-Gehalt der Zellkerne mit dem Durchflusszytometer gemessen und ausgewertet wurde (FACSCalibur und CellQuest Software; Becton Dickinson, Heidelberg). Aus den jeweils drei Messwerten wurde der Mittelwert berechnet, wobei die Messwerte der Kontrollzellen als Referenz für den t-Test dienten.

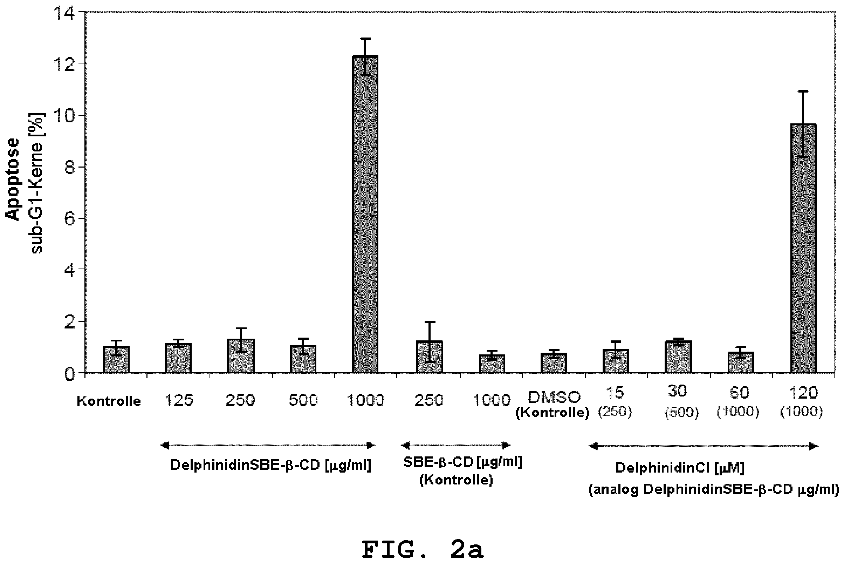

Es ist bekannt, dass bei Apoptosemessungen eine geringe spontane Apoptoserate (Hintergrundapoptose) auftreten kann, wobei dieser Effekt insbesondere dann auftritt, wenn Zellen bereits das Wachstumsplateau erreicht haben und an Nährstoff- und Sauerstoffmangel leiden. Um dies zu berücksichtigen wurde an Zellen gemessen, die sich in der exponentiellen Wachstumsphase befanden, wobei der Versuch parallel sowohl bei geringer (niedrige Zellkonfluenz), als auch höherer Zelldichte (höhere Zellkonfluenz) durchgeführt wurde, dessen Ergebnisse in den Figuren 2a (niedrige Zellkonfluenz) und 2b (höhere Zellkonfluenz) graphisch aufbereitet dargestellt sind.

### Versuchsergebnisse

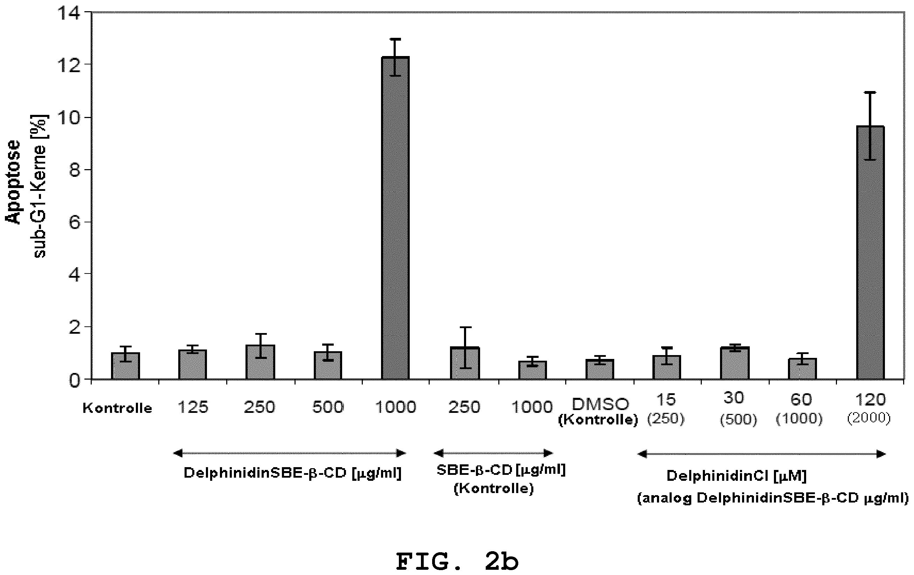

- Gereinigtes Delphinidin Cl zeigt dosisabhängig eine signifikante Apoptoseinduktion (vgl. Figur 2a bei 120 µM);
- Eine deutlich stärke Apoptoseinduktion zeigt der Delphinidin-SBE-β-CD-Komplex (vgl. Figur 2a bei 1000 µg/ml und Figur 2b bei 3000 µg/ml);
- Während der Komplexpartner SBE-β-CD sowie DMSO keine Wirkung zeigt, analog den unbehandelten Kontrollzellen.

### Beispiel 7

### (Zellviabilitätstest)

In dem Experiment gemäß Beispiel 7 wurde die Wirkung der untersuchten Substanzen auf die Zellviabilität der Testzelllinie mit Hilfe des WST-1-Assays (Water Soluble Tetrazolium) von Roche Diagnostics quantifiziert. Der WST-1-Assay dient zum Nachweis einer intakten Atmungskette in Zellen, wobei viable Zellen mit einem intakten mitochondrialen Succinat-Tetrazolium-Dehydrogenase-System eine enzymatische Umsetzung des schwach rot gefärbten Tetrazoliumsalzes WST-1 (4-[3-(4-Iodophenyl)-2-(4-nitrophenyl)-2H-5-tetrazolio]-1,3-Benzol-Disulfonat) in das dunkelrote Formazan bewirken. Dieser Farbumschlag kann in einem Spektralphotometer photometrisch gemessen und ausgewertet werden.

Für die Durchführung des WST-1-Assays gemäß Beispiel 7 wurden die Zellen analog Beispiel 6 für 24 Stunden mit 10-3000 µg Delphinidin-SBE-β-CD pro ml Zellsuspension bzw. 15-120 µM gereinigtes Delphinidin Cl pro ml Zellsuspension inkubiert, wobei mit dem Komplexpartner SBE-β-CD sowie DMSO behandelte Zellen, als auch unbehandelte Zellen als Kontrollen dienten. An den WST-1-Assay, wie beispielsweise in Plötz et al. (2012), Mutual Regulation of Bcl-2 Proteins Independent of the BH3 Domain as Shown by the BH3-Lacking Protein Bcl-xAK, PLOS ONE, vol 7, issue 4, e34549 im Einzelnen beschrieben, schlossen sich die Färbung mit Calcein-AM mittels Inkubation mit Calcein ( 4µM; e Bioscience, Frankfurt, Deutschland) in Serum-freien Wachstumsmedium für 60 Minuten bei 37°C sowie eine Waschung mit PBS an, bevor die Zellviabilitätsmessung mittels Durchflusszytometrie (s. Beispiel 5) erfolgte um Calcein-gefärbte (lebende) Zellen von ungefärbten (toten) Zellen zu unterscheiden.

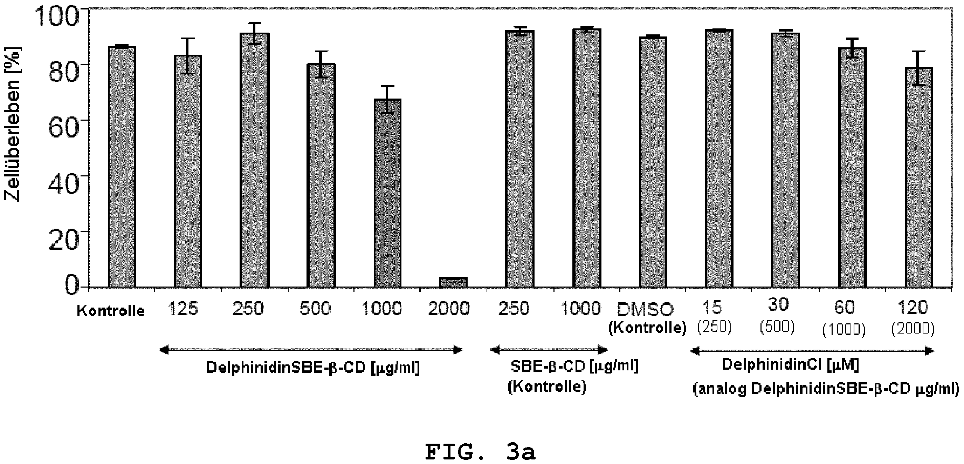

Analog Beispiel 6 wurde auch in Beispiel 7 der Versuch sowohl bei geringer (niedrige Zellkonfluenz), als auch höherer Zelldichte (höhere Zellkonfluenz) durchgeführt, dessen Ergebnisse in den Figuren 3a (niedrige Zellkonfluenz) und 3b (höhere Zellkonfluenz) graphisch aufbereitet dargestellt sind.

### Versuchsergebnisse

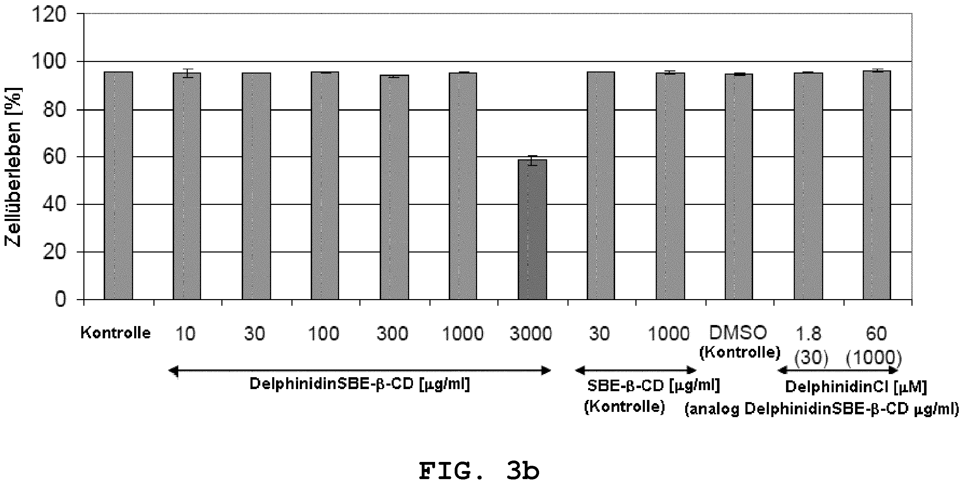

- Gereinigtes Delphinidin Cl zeigt mit steigender Dosis einen Verlust an vitalen Zellen bei niedriger Zellkonfluenz (vgl. Figur 3a);
- Eine deutlich stärkeren und signifikanten Verlust an Zellvitalität bewirkt der Delphinidin-SBE-β-CD-Komplex (vgl. Figur 3a bei 1000 µg/ml und 2000 µg/ml und Figur 3b bei 3000 µg/ml);
- Während der Komplexpartner SBE-β-CD sowie DMSO keine Wirkung zeigt, analog den unbehandelten Kontrollzellen.

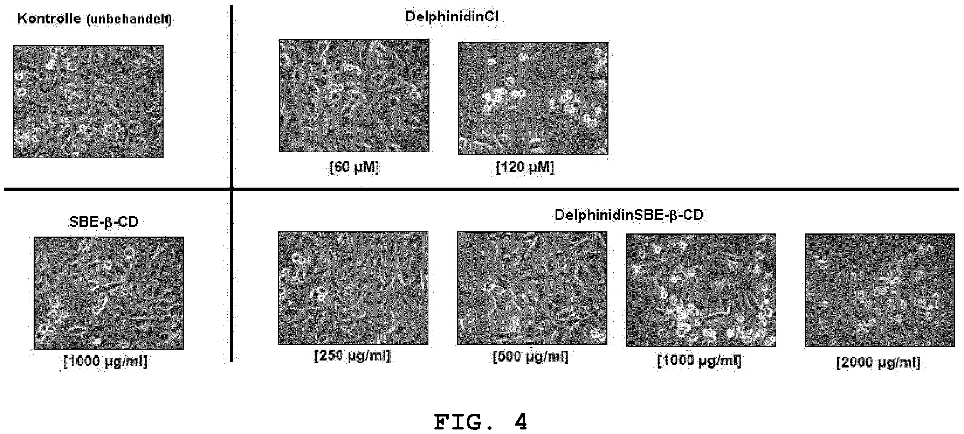

Der über die Apoptoseinduktion (Beispiel 6) hinausgehende Effekt des Verlustes an vitalen Zellen spiegelt sich auch in der Morphologie der Zellen bei mikroskopischer Betrachtung 24 stündiger Behandlung wieder, die sich bei hohen Konzentrationen von Delphinidin Cl bzw. Delphinidin-SBE-β-CD abkugeln und/oder ablösen, wie aus dem mikroskopischen Aufnahmen in Figur 4 ersichtlich.

### Beispiel 8

### [Kontinuierliches Echtzeit-Zell-Monitoring (Real-time cell analysis - RTCA)]

Die Zellzahl und Zellproliferation mit gleicher Wirkstoffuntersuchung analog den Beispielen 6 und 7 wurde in Echtzeit mittels des Systems xCELLLigence (Roche Diagnostics, Mannheim, Deutschland) aufgezeichnet. Das xCELLigence System ist ein mikroelektronisches Biosensorsystem für zellbasierte, markierungsfreie Analysen, das dynamische Zelldaten in Echtzeit liefert. Die Kulturplatten des xCELLigence Systems sind mit Mikroelektroden am Boden jeder Mulde ausgestattet, um Änderungen in der elektrischen Impedanz zu messen. Quantitative Impedanzänderungen korrelieren mit der Anzahl und Stärke der Zellkontakte mit den darunter liegenden Elektroden.

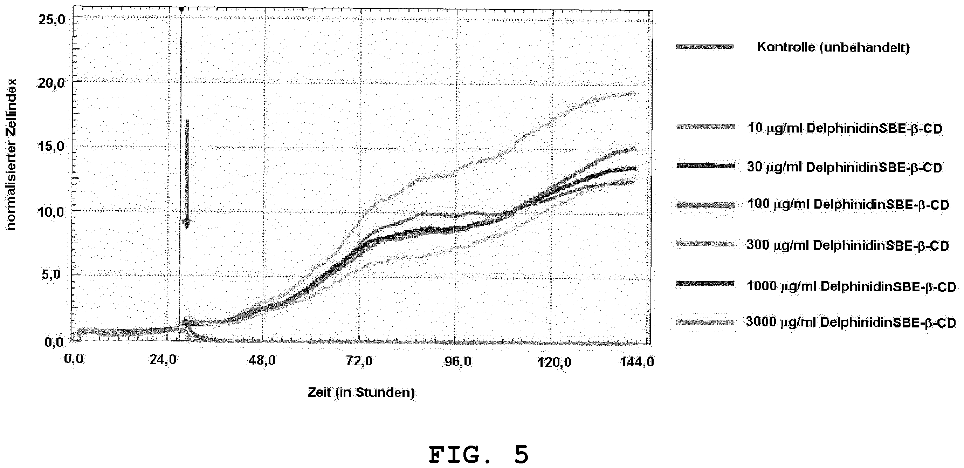

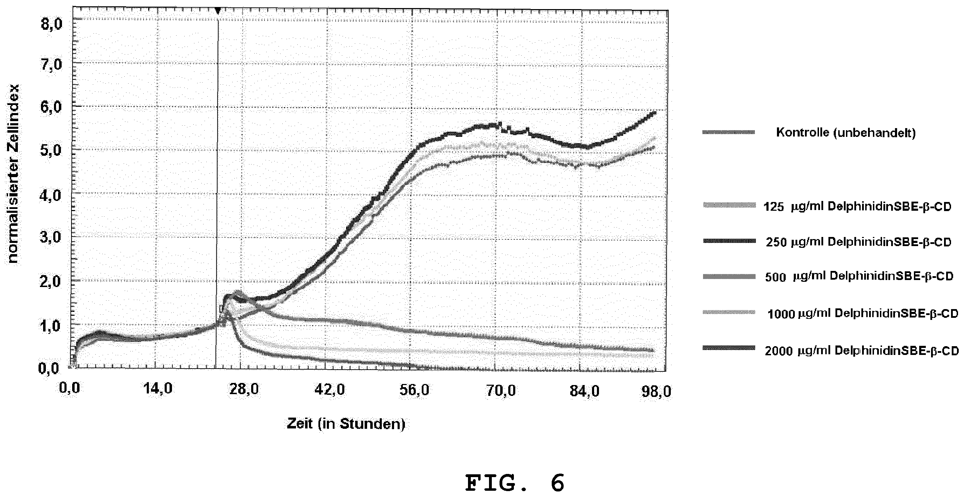

Delphinidin-SBE-β-CD zeigt in Übereinstimmung mit den hohen Apoptoseraten (Beispiel 6) sowie dem Verlust der Zellvitalität (Beispiel 7) einen vollständigen Einbruch der Zellproliferation (vgl. Figuren 5 und 6), insbesondere starke antiproliferative Effekte ab einer Konzentration von 1000 µg/ml in Figur 5 und eine vollständige Blockade der Zellproliferation ab 500 µg/ml in Figur 6.

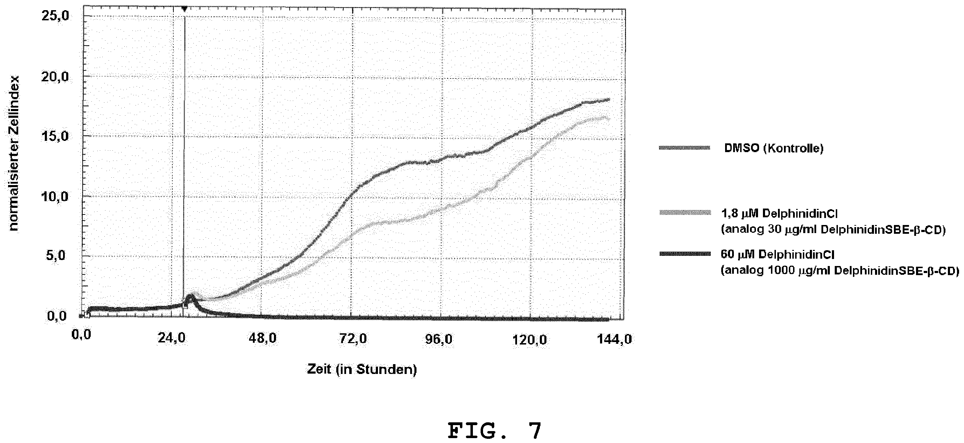

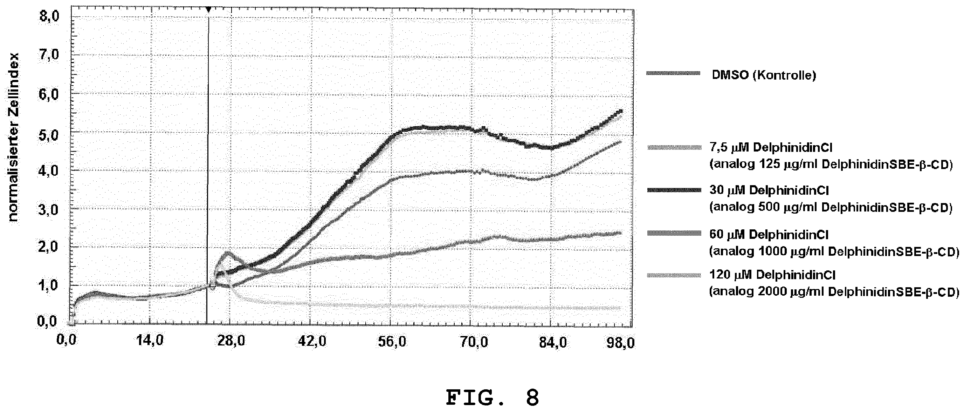

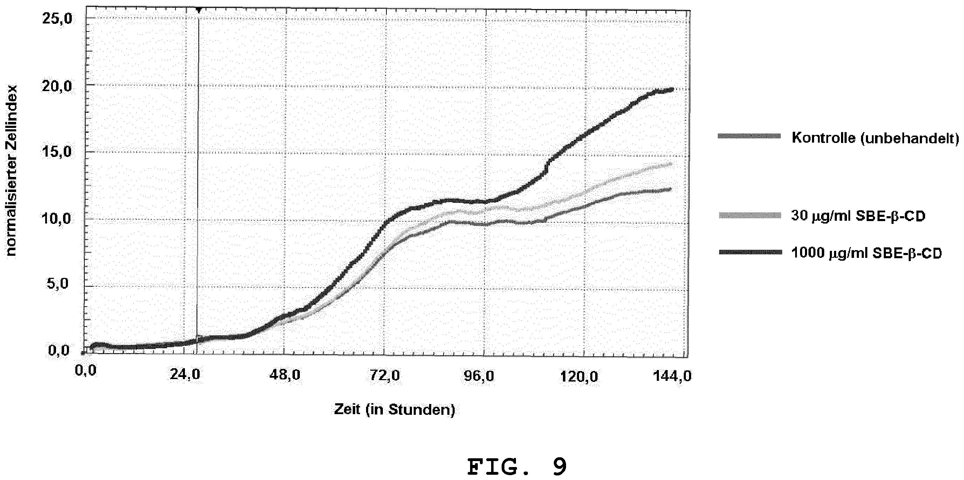

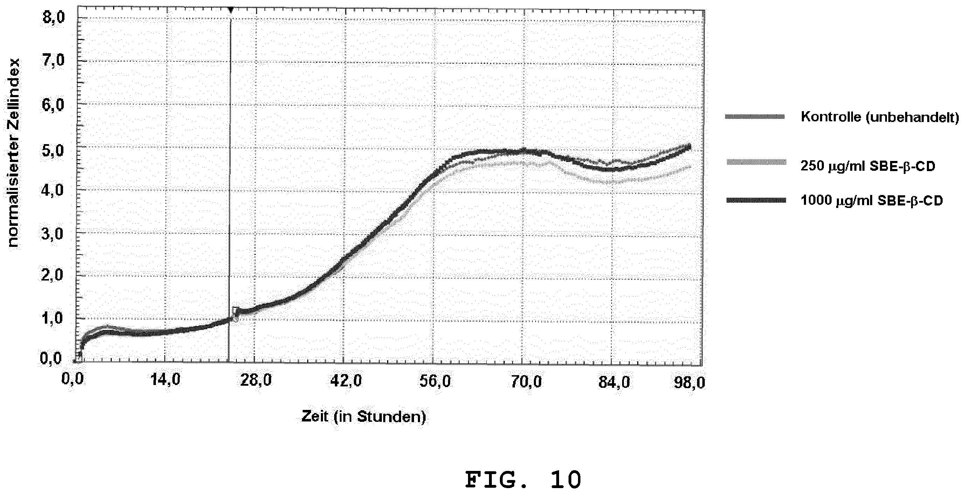

Ähnlich starke antiproliferative Effekte zeigen sich mit Delphinidin Cl bei 60-120 µM (vgl. Figuren 7 und 8), während sich erwartungsgemäß die unbehandelten Zellen sowie die DMSO-Kontrolle nahezu neutral verhielten (vgl. Figuren 7-10), d.h. die vorbeschriebenen antiproliferativen Wirkungen sind eineindeutig den untersuchten Wirkstoffen Delphinidin-SBE-β-CD und Delphinidin Cl zuzuschreiben.

### III. Wirkung von Delphinidin und des Delphinidin-SBE-β-CD-Komplexes in Kombination mit TRAIL auf Melanomzellen

### Testzelllinie und verwendete Verfahren

Bei den Versuchen wurde auf die gleiche Tellinie und Verfahren wie in vorangehendem Kapitel II verwendet.

### Beispiel 9

### (Apoptoseinduktionsmessung mit Hilfe der sub-G₁-Peak-Methode)

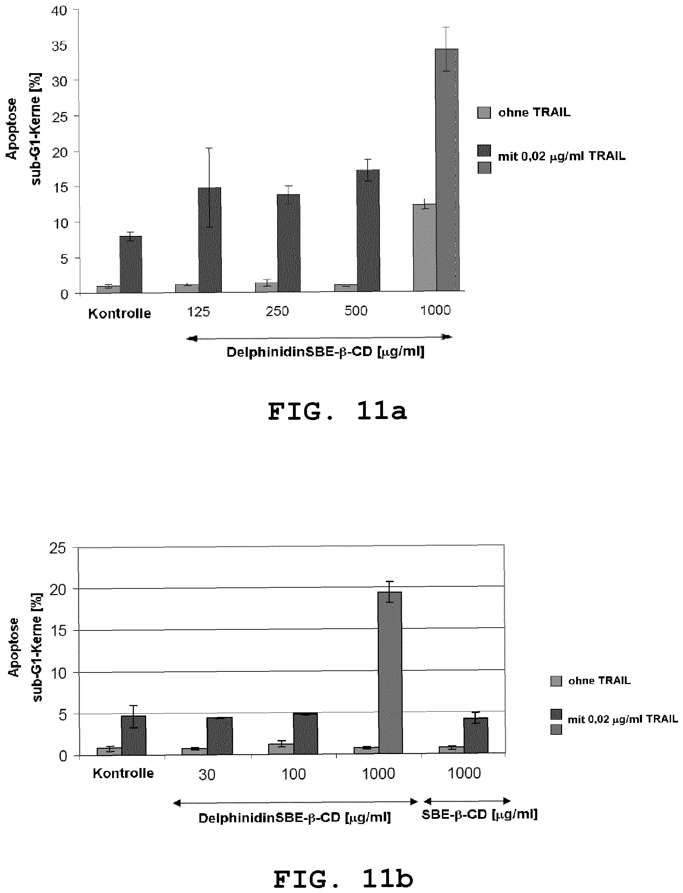

In dem Experiment gemäß Beispiel 9 wurde mittels der der sub-G₁-Peak-Methode die Wirkung des Delphinidin-SBE-β-CD Komplexes in Kombination mit dem proapoptotischen Todesliganden TRAIL untersucht, gegen den die Zelllinie A-375 nur moderate Apoptosesensitivität besitzt. Für die Durchführung der sub-G1-Peak-Methode gemäß Beispiel 9 wurden die Zellen für 24 Stunden mit 30-1000 µg Delphinidin-SBE-β-CD pro ml Zellsuspension mit und ohne 0,02 µg TRAIL pro ml Zellsuspension inkubiert, wobei mit dem Komplexpartner SBE-β-CD behandelte Zellen, als auch unbehandelte Zellen als Kontrollen dienten. Die Versuche wurden parallel sowohl bei geringer (niedrige Zellkonfluenz), als auch höherer Zelldichte (höhere Zellkonfluenz) durchgeführt, deren Ergebnisse in den Figuren 11a (niedrige Zellkonfluenz) und 11b (höhere Zellkonfluenz) graphisch aufbereitet dargestellt sind.

### Versuchsergebnisse

- Bei der Kombination von Delphinidin-SBE-β-CD mit TRAIL ist die Apoptoseinduktion gegenüber Delphinidin-SBE-β-CD allein bei allen untersuchten Delphinidin-SBE-β-CD-Konzentrationen gesteigert;
- Ebenfalls ist eine Steigerung der Apoptoseinduktion von Delphinidin-SBE-β-CD mit TRAIL gegenüber TRAIL allein bei höheren Delphinidin-SBE-β-CD-Konzentrationen signifikant gesteigert;
- Während der Komplexpartner SBE-β-CD auf Kontrollniveau liegt.

### Beispiel 10

### (Zellviabilitätstest)

In dem Experiment gemäß Beispiel 9 wurde analog Beispiel 7 die Zellviabilität der Testzelllinie unersucht, mit dem Unterschied, dass die Zellen der Kombination aus Delphinidin-SBE-β-CD Komplexe und TRAIL ausgesetzt wurden.

Für die Durchführung des Assays analog Beispiel 7 wurden die Zellen für 24 Stunden mit 30-1000 µg Delphinidin-SBE-β-CD pro ml Zellsuspension mit und ohne 0,02 µg/ml TRAIL pro ml Zellsuspension inkubiert, wobei dem Komplexpartner SBE-β-CD behandelte Zellen, als auch unbehandelte Zellen als Kontrollen dienten.

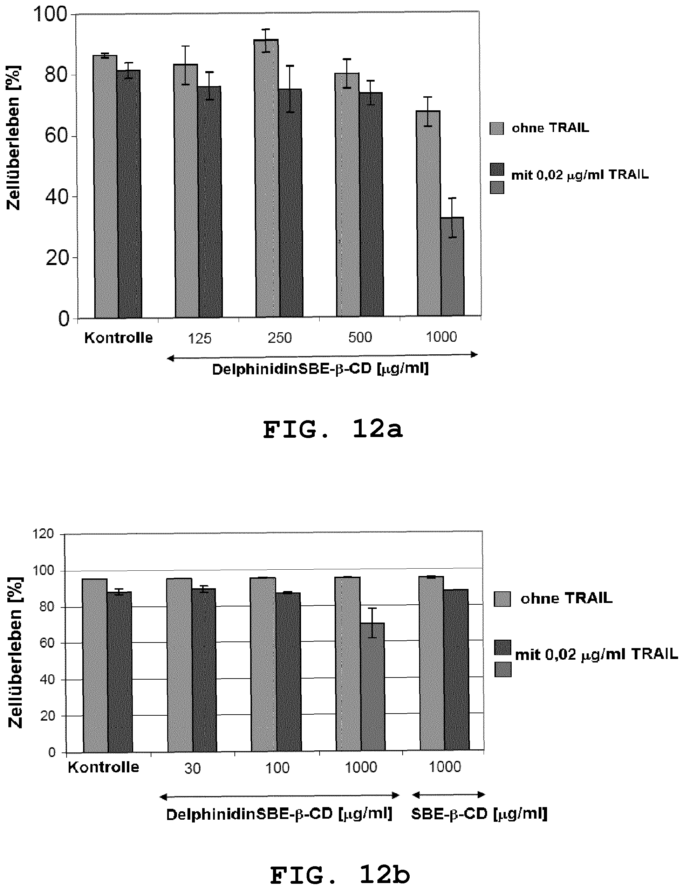

Analog Beispiel 9 wurde auch in Beispiel 10 der Versuch sowohl bei geringer (niedrige Zellkonfluenz), als auch höherer Zelldichte (höhere Zellkonfluenz) durchgeführt, dessen Ergebnisse in den Figuren 12a (niedrige Zellkonfluenz) und 12b (höhere Zellkonfluenz) graphisch aufbereitet dargestellt sind.

### Versuchsergebnisse

- Bei der Kombination von Delphinidin-SBE-β-CD mit TRAIL sinkt die Zellvitalität gegenüber dem Delphinidin-SBE-β-CD allein bei allen untersuchten Delphinidin-SBE-β-CD-Konzentrationen;
- Ebenfalls ist eine Absenkung der Zellvitalität die der Kombination von Delphinidin-SBE-β-CD mit TRAIL gegenüber TRAIL allein bei höheren Delphinidin-SBE-β-CD-Konzentrationen ersichtlich;
- Während der Komplexpartner SBE-β-CD auf Kontrollniveau liegt.

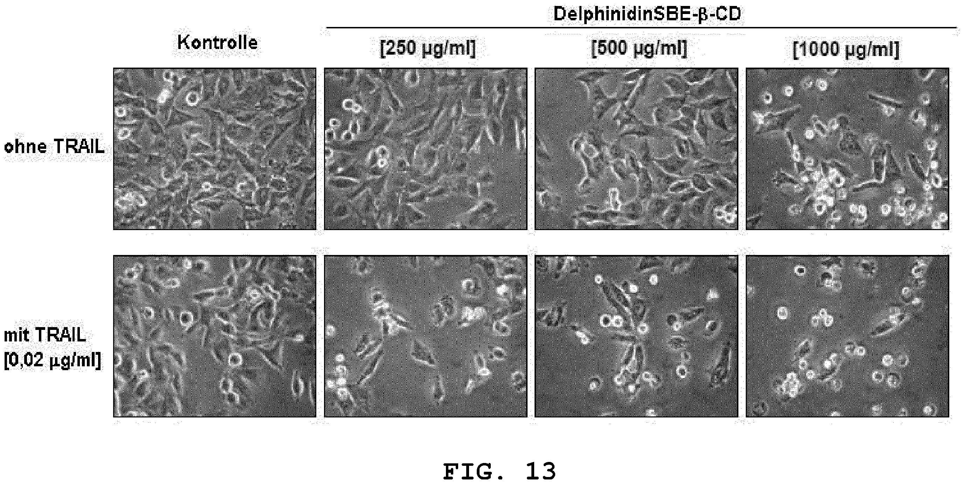

Der über die Apoptoseinduktion (Beispiel 9) hinausgehende Effekt des Verlustes an vitalen Zellen spiegelt sich auch in der Morphologie der Zellen bei mikroskopischer Betrachtung 24 Stunden nach der Behandlung wieder, die sich bei zusätzlicher Anwendung von TRAIL zu Delphinidin-SBE-β-CD noch stärker abkugeln bzw. ablösen, wie aus dem mikroskopischen Aufnahmen in Figur 13 ersichtlich.

### Beispiel 11

### [Kontinuierliches Echtzeit-Zell-Monitoring (Real-time cell analysis - RTCA)]

Die Zellzahl und Zellproliferation mit gleicher Wirkstoffuntersuchung wurde analog den Beispielen 9 und 10 und zusätzlich ergänzt um die Kombination aus Delphinidin Cl mit und ohne TRAIL in Echtzeit mittels des Systems xCELLLigence (Roche Diagnostics, Mannheim, Deutschland) aufgezeichnet.

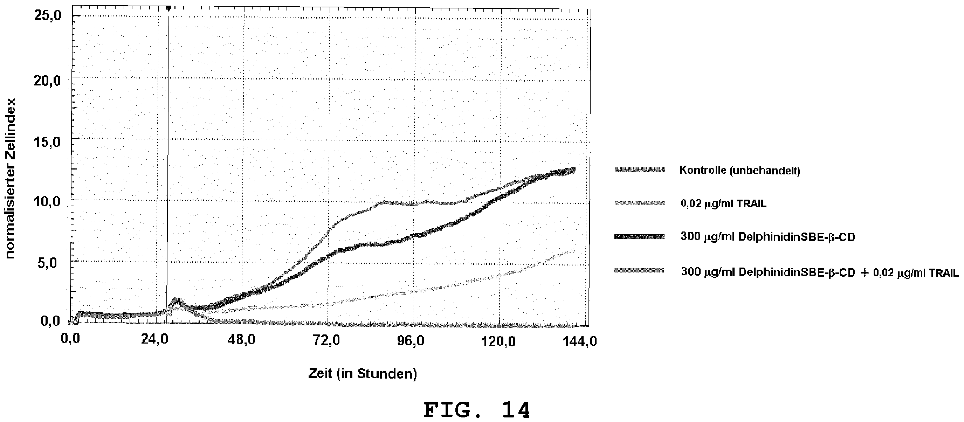

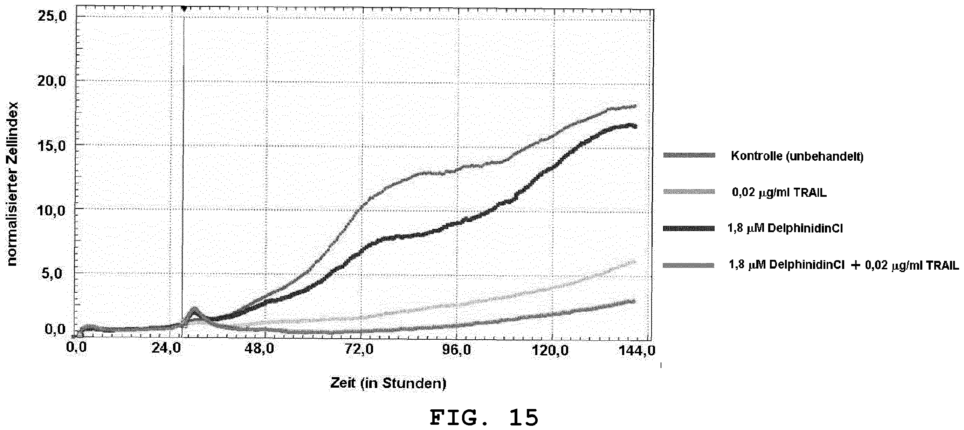

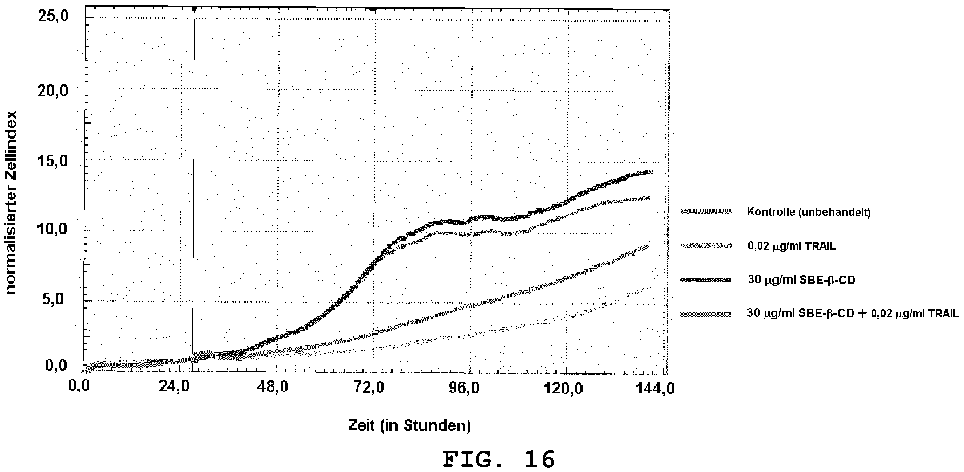

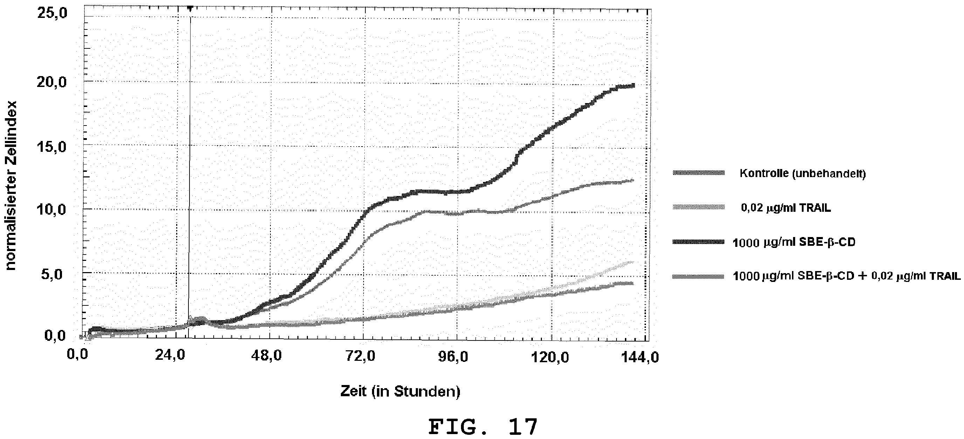

Delphinidin-SBE-β-CD zeigt bei Kombination mit TRAIL in Übereinstimmung mit den gegenüber Delphinidin-SBE-β-CD allein gesteigerten Apoptoseraten (Beispiel 9) sowie dem stärkeren Verlust der Zellvitalität (Beispiel 10) eine synergistische und stärkere Blockierung der Zellproliferation (vgl. Figur 14). Eine stärkere Blockierung der Zellproliferation lasst sich ebenfalls bei der Kombination von Delphinidin Cl mit TRAIL gegenüber TRAIL allein und Delphinidin allein feststellen (vgl. Figur 15). Keine Verstärkung der TRAIL-Sensitivität war durch SBE-β-CD zu beobachten (vgl. Figuren 16 und 17) d.h. die vorbeschriebenen antiproliferativen Wirkungsverstärkungen sind eineindeutig den untersuchten Wirkstoff Delphinidin im Delphinidin-SBE-β-CD in Kombination mit TRAIL zuzuschreiben.

### IV. Zytotoxizitätsuntersuchungen - Wirkung von Delphinidin und des Delphinidin-SBE-β-CD Komplexes auf die Vitalität von Zellen

### Beispiel 12

### (Wirkung von Delphinidin und des Delphinidin-SBE-β-CD Komplexes auf die Vitalität von humanen Fibroblasten und Endothelzellen)

### Verwendete Zellen

Bei den verwendeten Zellen wurde auf primäre, selbst isolierte Fibroblasten und mikrovaskuläre Endothelzellen aus humaner Spenderhaut zurückgegriffen.

Für die Isolierung der Fibroblasten wurde der Zellverband des Gewebes durch eine einstündige Inkubation mit Trypsin-EDTA-Lösung gelockert. Um die Ablösungsreaktion der Zellen zu beenden, wurde zur Haut Stoppmedium hinzu gegeben. Darauf wurde das Hautstück noch zweimal in PBS geschwenkt, so dass eine Fibroblastensuspension erhalten wurde. Die gewonnenen Fibroblasten wurden bei 4°C über 5 min mit 1000 Umdrehungen abzentrifugiert und nach Quantifizierung verwendet.

Die Gewinnung der humanen dermalen mikrovaskuläre Endothelzellen erfolgte mittels der dem Fachmann bekannten Standardmethode von Hewett and Murray (1993) [Hewett P.W. and Murray J.C. (1993) Immunomagnetic purification of human microvessel endothelial cells using Dynabeads coated monoclonal antibodies to PECAM-1. Eur J Cell Biol 62: 451-454; Hewett P.W. & Murray J.C. (1993) Human microvessel endothelial cells: Isolation, culture and characterization. In Vitro Cell Biol: 823-830].

### Verwendete Messverfahren

In den Experimenten gemäß Beispiel 12 wurde die Wirkung der untersuchten Substanzen auf die Vitalität von humanen Fibroblasten und Endothelzellen mit Hilfe des ATP-Lumineszenz-Assays untersucht, die dem Fachmann bekannt ist und nachfolgend der Vollständigkeit halber kurz zusammengefasst ist.

Nach dem Zelltod, beispielsweise aufgrund der Einwirkung eines zytotoxischen Wirkstoffs, nimmt der intrazellulare ATP-Gehalt durch ATPasen vermittelte Degradation im starken Maße ab. Bei Inhibierung der endogenen ATPasen kann der durch Lyse freigesetzte ATP-Gehalt folglich als Maß für die Anzahl vitaler Zellen verwendet werden. Für die Quantifizierung wird das freigesetzte ATP an eine von dem Enzym Luciferase katalysierte Reaktion gekoppelt, bei der in Gegenwart vom Mg²⁺ die ATP-abhängige Oxidation des Substrats Luciferin zu Oxyluciferin, Kohlendioxid, AMP, anorganisches Phosphat unter Freisetzung von Licht stattfindet. Die Menge des mit dem ATP-Gehalt korrelierten freigesetzten Lichtes wird gemessen und lässt einen mittelbaren quantifizierbaren Rückschluss auf die Vitalität der untersuchten Zellen zu. Gemessen wird in RLU (Relative Light Units), einer eigenständigen Einheit, die die Menge an ATP in der Probe widerspiegelt. Kits und Geräte für die ATP-Bestimmung sind dem Fachmann bekannt, beispielsweise der ATP-Test-Kit der Fa. Biothema AB, Haning, Schweden, und das tragbare Luminometer Lumino® der Fa. STRATEC Biomedical Systems AG, Birkenfeld, Deutschland. Zur Bestimmung des intrazellulären ATP werden üblicherweise 50 µl der zu untersuchenden Probe in ein Glasröhrchen pippettiert, dazu 50 µl des Extractant-Reagenzes B/S zur Zell-Lyse und 400 µl des ATP-HS-Reagenzes hinzugefügt und nach anschließender Mischung das verschlossene Röhrchen in das Luminometer zur automatischen Messung und Auswertung der Lichtemission gegeben.

Im Vorfeld der Wirkstoffbehandlung und Anwendung des ATP-Lumineszenz-Assays wurden im vorliegendem Fall die humanen Fibroblasten- bzw. Endothelzellen mit Dulbecco's Modified Eagle's Medium (DMEM-Medium) auf 96-Well-Mikrotiterplatten ausgesät (5000 Zellen/Well) und für 3 Tage kultiviert. Nach dieser Anheftungs- und Wachstumsphase der Zellen im Brutschrank fand die Substanzbehandlung mit Zeitintervallen von Tag 1-Tag 4 (Endothelzellen) und 4h, 24h, 48h und 96h (Fibroblasten) statt, wofür jeweils in 3 Wells in 100µl DMEM-Medium gelöster Wirkstoff DelphinidinCl bzw. Delphinidin-SBE-β-CD Komplex in unterschiedlichen Konzentrationen (0.1 µM; 3,2 µM; 100 µM) hinzugefügt wurde. Als Referenzkontrolle wurden Zellen mit substanzfreiem Medium verwendet. Die Abnahme der Substanzen erfolgte nach 3 Stunden, gefolgt von einer Waschung mit Dulbecco's Phosphate Buffered Saline (PBS). Die Endpunktmessung erfolgte mittels des bereits zu Beispiel 7 erwähnten WST-1-Assays.

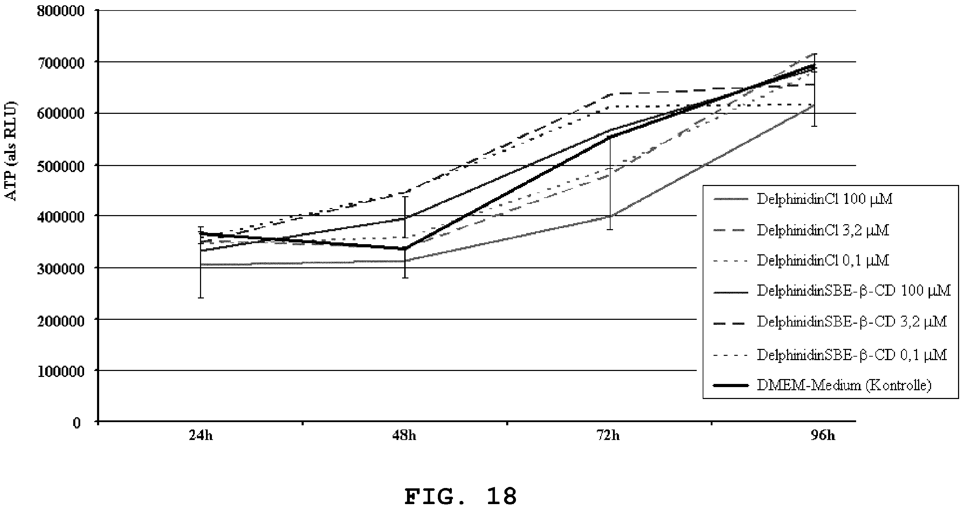

Die Versuchsergebnisse des ATP-Lumineszenz-Assays sind in den Figuren 18 (Endothelzellen) und 19 (Fibroblasten) graphisch aufbereitet und lassen sich wie folgt zusammenfassen:
- DelphinidinCl sowie Delphinidin-SBE-β-CD zeigen bei den untersuchten Wirkstoffkonzentration (0,1-100 µM keine signifikanten zytotoxischen Effekte auf humane Fibroblasten und Endothelzellen.

Damit erschließt sich eine völlig neue und besonders geeignete Wirkstoffgruppe zur Krebsbehandlung, weisen die untersuchten Wirkstoffe DelphinidinCl sowie Delphinidin-SBE-β-CD doch einerseits die erwünschten antiproliferative Effekte auf Krebszellen (Melanomzellen) auf (Beispiele 6-11), vermeiden aber anderseits die bei therapeutischen Wirkstoffkonzentrationen in der Regel zu erwartenden unerwünschten zytotoxischen (Neben-)Wirkungen auf Nicht-Krebszellen gemäß vorliegendem Beispiel 12.

## Patentansprüche

1. Zusammensetzung umfassend:
als eine erste Komponente
- einen Komplex aus Delphinidin und einem Sulfoalkylether-β-Cyclodextrin und/oder
- Delphinidin oder dessen Salze und
als zweite Komponente
- ein Tumornekrosefaktor-verwandter Apoptose-induzierter Ligand (Tumor Necrosis Factor Related Apoptosis Inducing Ligand - TRAIL)
zur Verwendung bei der Behandlung von malignem Melanom.

2. Zusammensetzung zur Verwendung nach Anspruch 1, **dadurch gekennzeichnet, dass** das Sulfoalkylether-β-Cyclodextrin im Komplex ein Sulfobutylether-β-Cyclodextrin ist.

3. Zusammensetzung zur Verwendung nach einem der vorangehenden Ansprüche, **dadurch gekennzeichnet, dass** im Komplex der Substitutionsgrad des Cyclodextrins mit Sulfoalkylethergruppen 3 bis 8, vorzugsweise 4 bis 8, weiter vorzugsweise 5 bis 8, weiter vorzugsweise 6 bis 7 beträgt.

4. Zusammensetzung zur Verwendung nach einem der vorangehenden Ansprüche, **dadurch gekennzeichnet, dass** die Zusammensetzung eine therapeutisch wirksame Menge an Delphinidin oder dessen Salzen oder Komplex aus Delphinidin und Sulfoalkylether-β-Cyclodextrin umfasst.

5. Zusammensetzung zur Verwendung nach einem der Ansprüche, **dadurch gekennzeichnet, dass** die Zusammensetzung mindestens eine weitere therapeutisch aktive Substanz umfasst.

6. Zusammensetzung zur Verwendung nach Anspruch 5, **dadurch gekennzeichnet, dass** die therapeutisch aktive Substanz ausgewählt ist aus der Gruppe bestehend aus
- Zytostatika,
- Interferone, vorzugsweise Alpha- und/oder Beta-Interferone, weiter vorzugsweise Interferon alpha 2-a und/oder alpha 2-b, und
- Tumorvakzine.

7. Zusammensetzung zur Verwendung nach einem der vorangehenden Ansprüche, weiter umfassend ein oder mehrere pharmazeutische Hilfs- und/oder Zusatzstoffe, vorzugsweise ausgewählt aus der Gruppe bestehend aus einen pharmazeutisch akzeptablen Träger, Füllstoffe, Geruchsstoffe und Stabilisatoren.

8. Zusammensetzung zur Verwendung nach einem der vorangehenden Ansprüche in einer Formulierungsform zur Verabreichung in einer Form ausgewählt aus der Gruppe bestehend aus oral, rektal, parenteral, einschließlich intraperitoneal, perkutan, subkutan, intramuskulär, intravenös, ophthalmisch, pulmonal und nasal.

9. Zusammensetzung zur Verwendung nach Anspruch 8, **dadurch gekennzeichnet, dass** die Verabreichungsform ausgewählt ist aus der Gruppe bestehend aus Tablette, Kapsel, Suspension, Aerosol, Lösung, Creme, Paste, Lotion, Gel und Salbe.

10. Zusammensetzung zur Verwendung nach einem der vorangehenden Ansprüche, **dadurch gekennzeichnet, dass** das Delphinidin oder dessen Salze und/oder der Komplex aus Delphinidin und dem Sulfoalkylether-β-Cyclodextrin in einer galenischen Zubereitung zur kontrollierten und/oder zeitverzögerten Freisetzung des Delphinidins eingesetzt wird.

11. Zusammensetzung zur Verwendung nach einem der vorangehenden Ansprüche, **dadurch gekennzeichnet, dass** die Behandlung des malignem Melanoms bei einem Objekt erfolgt, das einer Behandlung ausgewählt aus der Gruppe bestehend aus
- chirurgischer Entfernung von Melanomzellen oder von mit malignem Melanom befallenen Gewebe,
- Strahlentherapie,
- Immuntherapie,
- Chemotherapie und
- Interferon-Behandlung,
unterzogen wurde, unterzogen wird oder darauf vorbereitet wird.

12. Zusammensetzung zur Verwendung nach einem der vorangehenden Ansprüche, **dadurch gekennzeichnet, dass** die erste Komponente und die zweite Komponente Bestandteile eines Arzneimittels sind.

13. Zusammensetzung zur Verwendung nach einem der vorangehenden Ansprüche, **dadurch gekennzeichnet, dass** die erste Komponente und die zweite Komponente zur gleichzeitigen Verabreichung vorgesehen sind.

## Claims

1. Composition comprising:
as a first component
- a complex of delphinidin and a sulfoalkyl ether β-Cyclodextrin and/or
- delphinidin or salts thereof and
as second component
- a Tumor necrosis factor-Related Apoptosis-Inducing Ligand (TRAIL)
for use in the treatment of malignant melanoma.

2. Composition for use according to Claim 1, **characterized in that** the sulfoalkyl ether β-cyclodextrin in the complex is a sulfobutyl ether β-cyclodextrin.

3. Composition for use according to any of the preceding claims, **characterized in that** the degree of substitution of the cyclodextrin with sulfoalkyl ether groups in the complex is 3 to 8, preferably 4 to 8, more preferably 5 to 8, more preferably 6 to 7.

4. Composition for use according to any of the preceding claims, **characterized in that** the composition comprises a therapeutically active amount of delphinidin or salts thereof or complex of delphinidin and sulfoalkyl ether β-cyclodextrin.

5. Composition for use according to any of the claims, **characterized in that** the composition comprises at least one further therapeutically active substance.

6. Composition for use according to Claim 5, **characterized in that** the therapeutically active substance is selected from the group consisting of
- cytostatics,
- interferons, preferably alpha- and/or beta-interferons, more preferably interferon alpha-2a and/or alpha-2b, and
- tumor vaccines.

7. Composition for use according to any of the preceding claims, further comprising one or more pharmaceutical auxiliaries and/or additives, preferably selected from the group consisting of a pharmaceutically acceptable carrier, fillers, odorants and stabilizers.

8. Composition for use according to any of the preceding claims in a formulation form for administration in a form selected from the group consisting of oral, rectal, parenteral, including intraperitoneal, percutaneous, subcutaneous, intramuscular, intravenous, ophthalmic, pulmonary and nasal.

9. Composition for use according to Claim 8, **characterized in that** the administration form is selected from the group consisting of tablet, capsule, suspension, aerosol, solution, cream, paste, lotion, gel and salve.

10. Composition for use according to any of the preceding claims, **characterized in that** the delphinidin or salts thereof and/or the complex of delphinidin and the sulfoalkyl ether β-cyclodextrin are used in a galenic preparation for controlled and/or delayed release of the delphinidin.

11. Composition for use according to any of the preceding claims, **characterized in that** the treatment of malignant melanoma is carried out in a subject who has been subjected to, is being subjected to or is being prepared for a treatment selected from the group consisting of
- surgical removal of melanoma cells or tissues affected with malignant melanoma,
- radiotherapy,
- immunotherapy,
- chemotherapy and
- interferon treatment.

12. Composition for use according to any of the preceding claims, **characterized in that** the first component and the second component are constituents of a medicament.

13. Composition for use according to any of the preceding claims, **characterized in that** the first component and the second component are intended for simultaneous administration.

## Revendications

1. Composition comprenant :
comme premier composant
- un complexe de delphinidine et d'une sulfoalkyléther-β-cyclodextrine et/ou
- de la delphinidine ou ses sels et
comme deuxième composant
- un ligand apparenté au facteur de nécrose tumorale, induit par l'apoptose (Tumor Necrosis Factor Related Apoptosis Inducing Ligand - TRAIL)
pour une utilisation lors du traitement du mélanome malin.

2. Composition pour une utilisation selon la revendication 1, **caractérisée**
**en ce que** la sulfoalkyléther-β-cyclodextrine dans le complexe est une sulfobutyléther-β-cyclodextrine.

3. Composition pour une utilisation selon l'une quelconque des revendications précédentes, **caractérisée en ce que**, dans le complexe, le degré de substitution de la cyclodextrine par des groupes sulfoalkyléther est de 3 à 8, de préférence de 4 à 8, plus préférablement de 5 à 8, encore plus préférablement de 6 à 7.

4. Composition pour une utilisation selon l'une quelconque des revendications précédentes, **caractérisée en ce que** la composition comprend une quantité thérapeutiquement active de delphinidine ou de ses sels ou de complexe de delphinidine et de sulfoalkyléther-β-cyclodextrine.

5. Composition pour une utilisation selon l'une quelconque des revendications, **caractérisée en ce que** la composition comprend au moins une autre substance thérapeutiquement active.

6. Composition pour une utilisation selon la revendication 5, **caractérisée**
**en ce que** la substance thérapeutiquement active est choisie dans le groupe constitué par :
- les cytostatiques,
- les interférons, de préférence l'alpha-interféron et/ou le bêta-interféron, plus préférablement l'interféron alpha 2-a et/ou alpha 2-b, et
- les vaccins tumoraux.

7. Composition pour une utilisation selon l'une quelconque des revendications précédentes, comprenant en outre un ou plusieurs adjuvants et/ou additifs pharmaceutiques, de préférence choisis dans le groupe constitué par un support, des charges, des parfums et des stabilisants pharmaceutiquement acceptables.

8. Composition pour une utilisation selon l'une quelconque des revendications précédentes dans une forme de formulation pour une administration sous une forme choisie constituée par les formes orale, rectale, parentérale, y compris intrapéritonéale, percutanée, sous-cutanée, intramusculaire, intraveineuse, ophtalmique, pulmonaire et nasale.

9. Composition pour une utilisation selon la revendication 8, **caractérisée**
**en ce que** la forme d'administration est choisie dans le groupe constitué par un comprimé, une capsule, une suspension, un aérosol, une solution, une crème, une pâte, une lotion, un gel et une pommade.

10. Composition pour une utilisation selon l'une quelconque des revendications précédentes, **caractérisée en ce que** la delphinidine ou ses sels et/ou le complexe de delphinidine et de la sulfoalkyléther-β-cyclodextrine est/sont utilisé(s) dans une préparation galénique pour la libération contrôlée et/ou retardée dans le temps de la delphinidine.

11. Composition pour une utilisation selon l'une quelconque des revendications précédentes, **caractérisée en ce que** le traitement du mélanome malin a lieu sur un sujet qui a été soumis, qui est soumis ou qui est préparé à une soumission à un traitement choisi dans le groupe constitué par
- une élimination chirurgicale de cellules du mélanome ou d'un tissu envahi par un mélanome malin,
- une radiothérapie,
- une immunothérapie,
- une chimiothérapie et
- un traitement par interféron.

12. Composition pour une utilisation selon l'une quelconque des revendications précédentes, **caractérisée en ce que** le premier composant et le deuxième composant sont des constituants d'un médicament.

13. Composition pour une utilisation selon l'une quelconque des revendications précédentes, **caractérisée en ce que** le premier composant et le deuxième composant sont prévus pour une administration simultanée.
